(19) Europäisches Patentamt / European Patent Office / Office européen des brevets

(11) **EP 2 748 616 B1**

(12) # EUROPEAN PATENT SPECIFICATION

(45) Date of publication and mention
of the grant of the patent:
**08.08.2018   Bulletin 2018/32**

(51) Int Cl.:
*G01N 33/86* (2006.01)        *C12Q 1/56* (2006.01)
*G01N 33/49* (2006.01)        *C07K 14/81* (2006.01)

(21) Application number: **12750884.4**

(22) Date of filing: **20.08.2012**

(86) International application number:
**PCT/NL2012/050580**

(87) International publication number:
**WO 2013/028069 (28.02.2013 Gazette 2013/09)**

(54) **THERMOSTABLE INHIBITORS OF ACTIVATION OF THE BLOOD CLOTTING SYSTEM THROUGH CONTACT WITH FOREIGN SURFACES.**

WÄRMESTABILE HEMMER DER AKTIVIERUNG DES BLUTGERINNUNGSSYSTEMS DURCH KONTAKT MIT FREMDSUBSTANZEN

INHIBITEURS THERMOSTABLES D'ACTIVATION DU SYSTÈME DE COAGULATION DU SANG AU CONTACT DE SURFACES ÉTRANGÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **23.08.2011   EP 11178517**
       **23.08.2011   US 201161526368 P**

(43) Date of publication of application:
**02.07.2014   Bulletin 2014/27**

(73) Proprietors:
• **Synapse B.V.**
  **6229 ER Maastricht (NL)**
• **Universiteit Maastricht**
  **6211 LK  Maastricht (NL)**

(72) Inventors:
• **HACKENG, Tilman Mathias**
  **NL-6267 BG Cadier en Keer (NL)**
• **SUIJLEN, Dennis Peter Leonardo**
  **NL-6118 GH Nieuwstadt (NL)**
• **HEMKER, Hendrik Coenraad**
  **NL-6211 LM Maastricht (NL)**
• **APITZ-CASTRO, Rafael Jesus**
  **San Bernardino**
  **Caracas 1010 (VE)**

(74) Representative: **Nederlandsch Octrooibureau**
  **P.O. Box 29720**
  **2502 LS The Hague (NL)**

(56) References cited:
EP-A2- 0 252 057        WO-A1-2006/066878
WO-A1-2011/005598       WO-A1-2011/036444
WO-A2-2007/149542       US-A1- 2003 064 414
US-A1- 2006 035 816     US-B1- 6 403 381

• WIECZOREK M ET AL: "The squash family of serine proteinase inhibitors. Amino acid sequences and association equilibrium constants of inhibitors from squash, summer squash, zucchini, and cucumber seeds", BIOCHEMICAL AND BIOPHYSICAL RESEARCH COMMUNICATIONS, ACADEMIC PRESS INC. ORLANDO, FL, US, vol. 126, no. 2, 31 January 1985 (1985-01-31), pages 646-652, XP024835629, ISSN: 0006-291X, DOI: 10.1016/0006-291X(85)90233-5 [retrieved on 1985-01-31]
• J Leluk ET AL: "Preparation and characteristics of trypsin inhibitors from the seeds of squash (Cucurbita maxima) and zucchini (Cucurbita pepo var. Giromontia)", Acta biochimica Polonica, 1 January 1983 (1983-01-01), pages 127-138, XP055302541, POLAND Retrieved from the Internet: URL:http://www.actabp.pl/pdf/2_1983/127.pd f [retrieved on 2016-09-14]

**Description**

**FIELD OF THE INVENTION**

The present invention relates to the field of blood clotting. Specifically, the invention relates to particular inhibitors of artificial activation of the blood clotting process through contact with foreign surfaces.

**BACKGROUND OF THE INVENTION**

The clotting system of the blood is essential for stopping bleeding but also is instrumental in the development of thrombosis. Equally many people die from bleeding or thrombosis as from cancer. To be able to detect over-activity of the clotting system (that fosters thrombosis) or under-activity (which causes a bleeding tendency), therefore is of paramount medical importance. Such function testing is an ex vivo laboratory procedure that has to be executed on blood taken from a blood vessel via a needle into a vial. This necessarily implies contact of the blood with "foreign" surfaces, i.e. surfaces other than the normal inside of the blood vessels.

As soon as the blood comes into contact with such surfaces the clotting process is set into motion. Blood clotting factor XII (FXII) or Hageman factor adsorbs onto the foreign surface and thereby turns into an active enzyme that, with the help of other plasma proteins (high molecular weight kininogen and prekallikrein) activates factor XI, which in its turn activates factor IX. If care is taken to take the blood into a solution that contains a substance that binds free $Ca^{2+}$ ions (e.g. Na-citrate or EDTA) no further reaction takes place. FIXa can continue the process only if such ions are available.

Taking blood in citrate therefore is a standard procedure if blood is to be collected for the study of the clotting system. Nevertheless in such blood, factors XII, XI and IX are likely to be already activated to a greater or lesser extent.

The part of the thrombin generation process up to activation of FIX is called the contact activation system. The contact activation process as such is not a part of the natural thrombin generation system, but FXI and FIX do have a role in this natural system and need to remain non-activated if its function is to be probed correctly. Prior involuntary activation interferes with assessment of the natural thrombin generating capacity and with any other type of measurement of the blood clotting system in which factors XI and IX play a role or where, on the contrary, such role is to be excluded.

It therefore is necessary to inhibit the contact activation system. Because the contact activation process starts as soon as the blood is taken from the patient and meets a foreign surface, such as the inside of the needle but especially the inside of the blood collection tube. This is why the inhibitor has preferably to be added to the mixture in the tube in which the blood is collected. Such mixture will contain citrate or another $Ca^{2+}$-chelating substance and any other addition that may be required.

By adding such inhibitor of contact activation the blood remains in its native state and the natural thrombin generation (TG) process and its mechanisms or a relevant part thereof can then be studied after recalcification.

For being tested, the entire thrombin generation process is best triggered in the same way as it is under natural circumstances in a wound or thrombus, i.e. by tissue factor. When measuring thrombin activity in a sample comprising a $Ca^{2+}$ chelating substance, sufficient $Ca^{2+}$ has to be added to enable the blood clotting cascade.

Tissue factor is a membrane protein that occurs on perivascular cells that are only exposed to blood when the vessel wall is damaged. It also occurs in inactive form ("encrypted") in certain white blood cells. It can be "decrypted" when these cells are activated by processes that normally occur during bacterial infection, such as exposure to lipopolysaccharides or other products from contaminating bacteria. For this reason, and also because of general medical and hospital hygiene, it is necessary that the tubes on which blood is taken are sterile to avoid aforementioned processes that would severely influence outcome of laboratory coagulation tests. An inhibitor of the contact activation system therefore should remain active during sterilization of the containers used for taking blood..

Thrombin generation is an extremely complicated web of enzymatical reactions. Its backbone is a series of proenzyme-enzyme reactions in which each proenzyme, once activated, activates the next one (the "clotting cascade").

The contact activation system is not a part of the natural cascade but the necessary inhibition of the contact activation system should not, as a side effect, inhibit any of the multiple reactions of the normal thrombin generation network, i.e. of thrombin generation as triggered by tissue factor.

Many of the reactions in this network occur at a phospholipid-solute (plasma) interface. Only phospholipids that are normally at the inside of a cell can serve this purpose. As a part of the clotting process of blood specific cells (blood platelets) turn their membranes inside-out so as to facilitate thrombin formation. Bacterial contamination can potentially cause blood platelets and white blood cells to expose procoagulant phospholipids, which is another way to disturb the natural clotting process. This is a second reason why an inhibitor of the contact activation system therefore should remain active during sterilization.

Corn trypsin inhibitor (CTI) from its natural source, is known for the purpose of inhibiting contact activation of blood (US6,403,381). CTI is heat labile and therefore has a short shelf-life and must be stored under special conditions. More important is that it cannot be sterilized without losing activity.

There is thus a need for a substance that inhibits contact activation and is heat resistant.

## DETAILED DESCRIPTION OF THE INVENTION

Surprisingly, it has now been demonstrated that specific thermostable peptides inhibit contact activation of blood, while not inhibiting thromboplastin induced thrombin generation, i.e. not impeding the normal clotting process.

Accordingly, in a first aspect the present invention provides a composition comprising at least one calcium chelating substance with blood clotting inhibition activity and further comprising a thermostable peptide of at most 42 amino acids which inhibits contact activation of the blood clotting system, said peptide comprising the consensus amino acid sequence: RVCPXILMXCKKDSDCLAECXCLEHGYCG (SEQ ID NO: 7), wherein X at position 5 is K or R or an analog of R, preferably R or an analog of R; X at position 9 is E or K, preferably K; and X at position 21 is I or V, preferably V , wherein an analog of Arg is selected from the group consisting of: monomethylarginine, symmetric and asymmetric dimethylarginine, canaline, 8-guanidino a-amino butyric acid, homoarginine, canavanine, and citrulline, and wherein said calcium-chelating substance is selected from citrate or a salt of citrate and is present in a concentration of 55mM to 260mM. The blood clotting system is known to the person skilled in the art, In short it is a sequence of proenzyme-enzyme conversions that for the most part occur at interfaces and that are controlled by positive and negative feedback reactions. It is succinctly described in the introduction of this application and in more detail in "The blood coagulation cascade" by Schenone M, Furie BC and Furie B. Current Opinion in Haematology 2004, Vol 11(4) pages 272-277.

Since a thermostable peptide used in the invention inhibits contact activation of the blood clotting process, it can conveniently be used in any biological fluid, including bodily fluids, including blood, where contact activation of the blood clotting system may occur. Blood may be freshly drawn blood, but may also be blood or a blood product such as plasma that has been stored.

A *calcium-chelating substance with blood clotting inhibition activity* is herein defined as any substance that binds free $Ca^{2+}$ ions while having blood clotting inhibition activity. By the binding of free $Ca^{2+}$, the blood clotting cascade is interrupted since factor IX cannot be activated in the absence of free $Ca^{2+}$ ions. Calcium-chelating substances with blood clotting activity are well known in the art and include e.g. ethylene-diamine-tetraacetic-acid (EDTA), citrate or salts of citrate, including Na-citrate. Preferably, the calcium chelating substance with blood clotting inhibition activity is compatible with measuring thrombin activity, a preferred substance is Na-citrate.

A *peptide* is herein defined as a polymer of amino acids linked by chemical bonds, preferably peptide bonds. In principle the amino acids may be any naturally occurring or unnatural or uncoded amino acids, or a mix thereof. A dipeptide consisting of two amino acids is the smallest peptide possible. A peptide may comprise from two to multiple thousand amino acids, e.g. 2, 3, 4, 5, 6 7, 8, 9, 10, 20, 30, 40, 50, 100, 200, 500, 800, 100, 1200, 1500, 2000 etc. amino acids.

*Inhibition* is herein defined as a reduction in an activity. The reduction may be any reduction, e.g. 1-fold, 10-fold, 100-fold, 1000-fold reduction.

*Contact activation* is herein defined as activation of the blood clotting process by contact of the blood with any other surface than its natural environment such as the inner surface of a vein. Such foreign surface may e.g. be a blood collection device or a hollow needle. The blood clotting process is explained earlier herein. It follows that *Inhibition of contact activation* is when contact activation is reduced.

*About 40 amino acids* is herein defined as approximately 40 amino acids, i.e. 38, 39, 40, 41, or 42 amino acids, preferably 40 amino acids.

*A position corresponding to amino acid position 5 in SEQ ID NO: 1* is herein defined as the position in a peptide according to the present invention when aligned to SEQ ID NO: 1 that corresponds to the position of the amino acid Arginine (R) at position 5 of SEQ ID NO: 5.

*Identity in the expression "A peptide which has at least 70% sequence identity with a peptide as shown in SEQ ID NO: 1, SEQ ID NO: 2, SEQ ID NO: 3, or SEQ ID NO: "* is defined as known in the art, as a relationship between two or more amino acid (peptide) sequences by comparing the sequences. In the art, the percentage of "identity" indicates the degree of sequence relatedness between amino acid as determined by the match between strings of such sequences. Preferably, the percentage of identity is determined by comparing the whole SEQ ID NO as identified herein. However, part of such sequence may also be used. In this context, "part" means at least 50%, 60%, 70%, 80%, 90% or 100% of its length. Preferably 100% is used. The sequence of the thermostable peptide to be used in the invention is as defined in appended claims 1, 4 or 5. Two amino acid sequences are considered "similar" if the polypeptides only differ in conserved amino acid substitutions. In determining the degree of amino acid similarity, the skilled person takes into account "conservative" amino acid substitutions. Conservative amino acid substitutions refer to the interchange of amino acids having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; and a group of amino acids having sulphur-containing side chains is cysteine and methionine. Preferred conservative amino acids substitution groups are: valine-leucine-isoleucine, phenylalanine-tyrosine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Asn to gln or his; Asp to glu; Cys to ser or ala; Gln to asn; Glu to asp; Gly to pro; His to asn or gln; Ile to leu or val; Leu to ile or val; Lys to arg; Asn to gln or glu; Met to leu or ile; Phe to met, leu or tyr; Ser to thr; Thr to ser; Trp to tyr; Tyr to trp or phe; and, Val to ile or leu. In the case of Arg, preferred conservative substitutions and analogs therefore are defined later herein.

"Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include *e.g.* the GCG program package (Devereux, J., et al., Nucleic Acids Research 12 (1):387 (1984)), BestFit and FASTA (Altschul, S. F. et al., J. Mol. Biol. 215:403-410 (1990). The BLAST 2.0 family of programs which can be used for database similarity searches includes: BLASTP for protein query sequences against protein database sequences. The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Another preferred method to determine sequence identity and similarity is by using the algorithm Needleman-Wunsch (Needleman, S. B. and Wunsch, C. D. (1970) J. Mol. Biol. 48, 443-453, Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The following website could be used: http://www.ebi.ac.uk/Tools/emboss/align/index.html. Definitions of the parameters used in this algorithm are found at the following website:: http://emboss.sourceforge.net/docs/themes/AlignFormats.html#id.Preferably, using this algorithm, Gap_penalty is 10.0 and Extend_penalty is 0.5.

*The amino acid Arg* is herein defined as the amino acid Arginine (R) or an analog of the amino acid Arginine; preferably the amino acid Arginine (R). *An analog of Arg* is selected from the group consisting of: monomethylarginine, symmetric and asymmetric dimethylarginine, canaline, $\delta$-guanidino $\alpha$-amino butyric acid, homoarginine, canavanine, citrulline. A preferred position where this definition applies is the Arginine at a position corresponding to amino acid position 5 in SEQ ID NO: 1.

In the composition according to the disclosure, the calcium-chelating substance with blood clotting inhibition activity and the thermostable peptide which inhibits contact activation of the blood clotting system may be present in the composition in any concentration and ratio as long as the composition still retains the activities of both compounds when in the final concentration in the blood, plasma or other biological fluid as described earlier herein. Preferably, the calcium chelating substance with blood clotting inhibition activity such as sodium citrate is present in such concentration that results in a final concentration from about 11 mM to 13 mM, more preferably 11 mM. In the composition of the invention, the calcium-chelating substance is selected from citrate or a salt of citrate and is present in a concentration of 55mM to 260mM. The thermostable peptide according to the invention which inhibits contact activation of the blood clotting system is preferably present in such concentration that results in a final concentration in blood of from about 5 $\mu$g/ml to 500 $\mu$g/ml, more preferably 10 $\mu$g/ml to 100 $\mu$g/ml. Preferably, when present in a sampling container, the calcium chelating substance with blood clotting inhibition activity such as sodium citrate is present in a stock solution of 3.2 or 3.8% in a sampling container resulting in a final concentration in blood from about 11 mM to 13 mM, more preferably 11 mM. Preferably, the thermostable peptide according to the invention which inhibits contact activation of the blood clotting system is present in a stock solution in the sampling tube resulting in a final concentration in blood of from about 10 $\mu$g/ml to 100 $\mu$g/ml. The stock solution may have any stock concentration, including 5, 10, 20-fold the preferred final concentration. Preferably, the stock solution has 10-fold the final concentration. The person skilled in the art will understand that when whole blood is drawn, the final concentration of the calcium chelating substance and of the thermostable peptide according to the invention will result in about twice these concentrations in plasma, depending upon the volume taken by red blood cells, which is around 50% of the total volume of blood. In all embodiments of the present invention, the final concentration of the thermostable peptide according to the invention which inhibits contact activation of the blood clotting system may be any concentration as long as the peptide still retains its inhibition activity; preferably the final concentration is from about 5 $\mu$g/ml to 500 $\mu$g/ml, more preferably 10 $\mu$g/ml to 100 $\mu$g/ml. The peptide used in the invention comprises at most 42, 41, 40, 39, 38, 37, 36, 35, 34, 33, 32, 31, 30, 29, 28, 27, 26, 25, 24, 23, 22, 21, 20 amino acids, more preferably between about 25-35 amino acids, more preferably about 27-32 amino acids, and most preferably about 29 amino acids.

When a peptide used in the invention is longer or shorter than the most preferred 29 amino acids, the net charge of the peptide is preferably substantially identical to the net charge of SEQ ID NO: 1 when measured under identical conditions, preferably under physiological conditions. Substantially identical in this context means that the net charge of the peptides is preferably within +0.5 and -0.5, more preferably within +0.4 and -0.4, within +0.3 and -0.3, within +0.2 and -0.2, within +0.1 and -0.1 of the net charge of SEQ ID NO: 1 when measured under identical conditions, preferably under physiological conditions. Most preferably, the net charge of the peptide is identical to the net charge of SEQ ID NO: 1 when measured under identical conditions, preferably under physiological conditions. Preferably, the pI of the peptide is substantially identical to the pI of SEQ ID NO: 1. Substantially identical pI means that the pI of the peptide is preferably within +0.5 and -0.5, more preferably within +0.4 and -0.4, within +0.3 and -0.3, within +0.2 and -0.2, within +0.1 and -0.1 of the pI of SEQ ID NO: 1.

The inhibition of contact activation is best measured by the prolongation of the activated partial thromboplastin time (aPTT). The aPTT is known to persons skilled in the art as a clotting time of plasma that is started by recalcification of citrated plasma in the presence of a substance that activates the contact system, such as kaolin or ellagic acid. It is described in detail by L.Poller in "Laboratory techniques in thrombosis" Edited by J.Jespersen, R.M.Bertina and F.Haverkate , ISBN 0-7923-5317-X (1999) p.37-44. Alternatively the prolongation of the lag time of a thrombin generation experiment according to WO2003093831 can be determined. One functional unit of a contact activator inhibitor is defined as the amount that doubles the aPTT of the lagtime of a thrombin generation curve.

The minimal desired degree of inhibition of contact activation is the one that triples the lag time of a TG curve or an a PTT measurement.

Preferably, thermostability with respect to the thermostable peptide used in the invention is such that no activity of inhibition of contact activation of the blood clotting system is lost under conditions of heat-sterilisation, i.e. 120°C for 20 minutes, as measured with a 0.1 mM solution of the thermostable peptide in a closed vial. However, in general a 50% loss of activity can be accepted as long as the minimal degree of inhibition is still attained after sterilisation.

In all the embodiments of the present invention, the thermostable peptide which inhibits contact activation of the blood clotting system may be present as a single peptide but different peptides may be present; i.e. in all embodiments according to the invention it is envisioned that the peptides may be mixed and applied in a mixture.

It will be evident that the composition according to the invention can conveniently be used for the collection of blood, to inhibit activation of the $Ca^{2+}$, non physiological part of the clotting reaction sequence, i.e. the contact system. Accordingly, in a second aspect the present invention provides a device for collecting a sample comprising a composition according to claim 1. Said device may be any device suitable for the collection or storage of a sample. Preferably, the sample is blood or blood plasma. If the sample is blood, the device is preferably a container for collection blood such as a tube, which may be a under vacuum in order to facilitate drawing blood from a subject. The device may also be a bag for collection of blood. Tubes (such as Becton Dickinson vacutainer glass, Sarstedt tubes; Terumo Venosafe, Becton Dickinson vacutainer plastic, Haemtech SCAT, Greiner vacuum tube) and bags for the collection of blood are known to the person skilled in the art.

The composition and device according to the invention can conveniently be used to prevent contact activated clotting of blood when determining blood clotting activity in blood. Contact activation would bias the determination and investigation, especially while determination blood clotting activity by measuring thrombin activity over time (thrombin generation). Accordingly, in a third aspect the present invention provides a kit of parts comprising:

- a device according to claim 2, and
- a reagent for determining blood clotting activity, preferably the development of thrombin activity as a function of time.

Preferably, in the kit according to the invention, the device for collecting a sample may be any device, but is preferably a container for collection blood such as a tube, which may be a under vacuum in order to facilitate drawing blood from a subject.

The kit may comprise any other reagent or device useful for the determination of blood clotting activity. The reagent for determining blood clotting activity may be any reagent known for that purpose to the person skilled in the art. When thrombin activity is measured, the reagent preferably is a fluorogenic thrombin substrate, preferably an oligopeptide coupled to a fluorogenic group. The general formula of such peptides is E-(AA)n-(Arg or Lys)-Fluo, wher E is an end group, AA an aminoacid and Fluo preferably aminomethylcoumarine or Rhodamine-110.

As described earlier herein, the thermostable peptide can conveniently be used to inhibit contact activation of the blood clotting system. Accordingly, in a fourth aspect the present invention provides the use of a thermostable peptide of at most 42 amino acids which inhibits contact activation of the blood clotting system, said peptide comprising the consensus amino acid sequence: RVCPXILMXCKKDSDCLAECXCLEHGYCG (SEQ ID NO: 7), , wherein X at position 5 is K or R or an analog of R, preferably R or an analog of R; X at position 9 is E or K, preferably K; and X at position 21 is I or V, preferably V wherein an analog of Arg is selected from the group consisting of: monomethylarginine, symmetric and asymmetric dimethylarginine, canaline, 8-guanidino a-amino butyric acid, homoarginine, canavanine, and citrulline, for *ex vivo* inhibiting activation of the blood clotting system through contact with foreign surfaces.

In the use according to the invention, the thermostable peptide which inhibits contact activation of the blood clotting system may be contacted with the blood or any other biological fluid or a blood product such as or plasma as described earlier herein, in every way available to the person skilled in the art. The blood is *ex vivo* blood or plasma, i.e. the taking of the blood has already taken place; the blood or plasma may have been stored. In the use according to the invention, the thermostable peptide which inhibits contact activation of the blood clotting system is preferably present upon first contact of the blood with the foreign surface or as soon as possible after contacting with the blood, in concentrations and ratio's as described in the first aspect of the invention. Preferably, the thermostable peptide is present together with a calcium chelating substance with blood clotting inhibition activity; the calcium-chelating substance with blood clotting inhibition activity and the thermostable peptide which inhibits contact activation of the blood clotting system are preferably present in concentrations and ratio's as described in the first aspect of the invention. Preferably, the thermostable peptide and the calcium-chelating substance with blood clotting inhibition activity are mixed with blood immediately after taking of blood in order to inhibit contact activation of the blood clotting system. This can conveniently be achieved by placing a thermostable peptide and the calcium chelating substance with blood clotting inhibition activity in the container wherein the blood is collected. As such, the blood can be mixed immediately after the blood sample has been drawn. The container is preferably a device as described in the second aspect of the invention.

The thermostable peptide can conveniently be used during the taking of a blood sample to inhibit contact activation of the blood clotting system, which clotting may otherwise already start during taking of the sample.

The blood clotting activity may be measured by any assay available to the person skilled in the art. Preferably, blood clotting activity is determined by measuring thrombin activity over time (thrombin generation). Thrombin activity may be measured by any means known to the person skilled in the art. A preferred assay uses a fluorogenic thrombin substrate, preferably an oligopeptide coupled to a fluorogenic group. The general formula of such peptides is E-(AA)n-(Arg or Lys)-Fluo, wher E is an end group, AA an aminoacid and Fluo preferably aminomethylcoumarine or Rhodamine-110 for measuring thrombin activity. Such assay is described in WO2003/093831 for plasma and in WO2006/117246 for whole blood. In a typical assay two parts of plasma or blood are mixed with one part of a solution containing at least $Ca^{2+}$, a fluorogenic substrate as described above and tissue factor so as to start thrombin generation.

The value of the blood clotting activity preferably is used for the diagnosis of several procoagulant and anticoagulant conditions, such as congenital or acquired thrombophilia, congenital or acquired hemophilia, consumption coagulopathy, or the effect of antithrombotic drugs (such as heparin, vitamin K antagonists, direct thrombin inhibitors) or procoagulant drugs (such as concentrates of factor VIII or IX). The normal value of the endogenous thrombin potential, i.e. the area under the thrombin generation curve, when tested by the method known to the art as examplified in example 1, 1600 $\pm$ 250 nM.min. Values below 400 nM.min indicate a definite bleeding tendency, values above 1850 nM.min a thrombotic tendency.

As described earlier herein, the thermostable peptide can conveniently be used to inhibit contact activation of the blood clotting system. Accordingly, in a fifth aspect the present invention provides an *ex vivo* method for inhibiting clotting of blood, the method comprising contacting a thermostable peptide of at most 42 amino acids which inhibits contact activation of the blood clotting system, said peptide comprising the consensus amino acid sequence: RVCPXILMXCKKDSDCLAECXCLEHGYCG (SEQ ID NO: 7), wherein X at position 5 is K or R or an analog of R, preferably R or an analog of R; X at position 9 is E or K, preferably K; and X at position 21 is I or V, preferably V, wherein an analog of Arg is selected from the group consisting of: monomethylarginine, symmetric and asymmetric dimethylarginine, canaline, 8-guanidino a-amino butyric acid, homoarginine, canavanine, and citrullin, with blood, plasma or any other biological fluid described earlier herein, in an amount sufficient to inhibit the clotting.

In the method for inhibiting clotting of blood according to the invention, the thermostable peptide which inhibits contact activation of the blood clotting system is contacted with the blood or other biological fluid as described earlier herein The thermostable peptide may be contacted with the blood in every way available to the person skilled in the art. After contacting with the blood, the peptide which inhibits contact activation of the blood clotting system is preferably present in an amount sufficient to inhibit clotting; preferably the thermostable peptide is present in concentrations and ratio's as described in the first aspect of the invention. Preferably, the thermostable peptide is present together with a calcium chelating substance with blood clotting inhibition activity; the calcium chelating substance with blood clotting inhibition activity and the thermostable peptide which inhibits contact activation of the blood clotting system are preferably present in concentrations and ratio's as described in the first aspect of the invention. The thermostable peptide may be contacted with the blood or other biological fluid as described earlier herein at any time. This may be performed immediately when taking the sample, but may also be performed later including after storage of the sample, e.g. before measurement of a parameter such as clotting activity.

As described earlier herein, a thermostable peptide can conveniently be used to inhibit contact activation of the blood clotting system. Therefore, said peptide can conveniently be used to collect and prepare a sample while inhibiting contact activation of the blood clotting system in said sample.

As described earlier herein, a thermostable peptide used in the invention inhibits contact activation of blood, while not inhibiting thromboplastin induced thrombin generation, i.e. not impeding the normal clotting process. Therefore, a thermostable peptide can conveniently be used in an assay wherein blood clotting activity is determined.

The blood clotting activity may be measured by any assay available to the person skilled in the art. Preferably, blood clotting activity is determined by measuring thrombin activity over time (thrombin generation). Thrombin activity may be measured by any means known to the person skilled in the art. A preferred assay uses a fluorogenic thrombin substrate, preferably aminomethylcoumarine for measuring thrombin activity. Such assay is described in WO2003/093831 for plasma and in WO2006/117246 for blood. See e.g. Example 1 herein.

According to the invention, in a composition according to the first aspect of the invention, a device according to the second aspect of the invention, a kit according to the third aspect of the invention, a use according to the fourth aspect of the invention, a method according to the fifth aspect of the invention, the thermostable peptide which inhibits contact activation of the blood clotting system is a thermostable peptide of at most 42 amino acids and comprising the consensus amino acid sequence: RVCPXILMXCKKDSDCLAECXCLEHGYCG (SEQ ID NO: 7), wherein X at position 5 is K or R or an analog of R, preferably R or an analog of R; X at position 9 is E or K, preferably K; and X at position 21 is I or V, preferably V, wherein an analog of Arg is selected from the group consisting of: monomethylarginine, symmetric and asymmetric dimethylarginine, canaline, 8-guanidino a-amino butyric acid, homoarginine, canavanine, and citrullin. According to the present disclosure (not part of the invention), the thermostable peptide which inhibits contact activation of the blood clotting system, preferably comprises a consensus amino acid sequence: XXCXXXXXXCXXXXXCXXXCXCXXXXXCX (SEQ ID NO: 9), wherein X at position 5 is R or an analog of R, ; X at position 9 is E or K, preferably K and X at position 21 is I or V, preferably V; or more preferably: XILMXCKKDSDCLAECX (SEQ ID NO: 6), wherein X at position 1 is K or R or an analog of R, preferably R or an analog of R; X at position 5 is E or K, preferably K and X at position 17 is I or V, preferably V; or more preferably: RVCPXILMXCKKDSDCLAECXCLEHGYCG (SEQ ID NO: 7), wherein X at position 5 is K or R or an analog of R, preferably R or an analog of R; X at position 9 is E or K, preferably K and X at position 21 is I or V, preferably V. In the aspect above defining SEQ ID NO:9, it is further preferred that SEQ ID NO:9 has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity with SEQ ID NO:1.

In an aspect (not part of the invention), the consensus amino acid sequence SEQ ID NO:9 comprises or consists of or is a consensus amino acid sequence: XXCXXXXXXCXXXXXCXXXCXCXXXXXCX,

- wherein X at position 5 is R or an analog of R,
- wherein X at position 1 is R or an analog or R,
- wherein X at position 2 is V or an analog of V,
- wherein X at position 9 is E or K, preferably K,
- wherein X at position 21 is I or V, preferably V, and/or
- wherein X at position 4 is P or an analog of P and preferably said SEQ ID NO:9 has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity with SEQ ID NO:1.

In this aspect, the consensus amino acid sequence may comprise or consist of:

**R**XCXXXXXXCXXXXXCXXXCXCXXXXXCX (SEQ ID NO:10)
X**V**CXXXXXXCXXXXXCXXXCXCXXXXXCX (SEQ ID NO:11)
XXC**P**XXXXXCXXXXXCXXXCXCXXXXXCX (SEQ ID NO:12)
XXCX**R**XXXXCXXXXXCXXXCXCXXXXXCX (SEQ ID NO:13)
**RV**CXXXXXXCXXXXXCXXXCXCXXXXXCX (SEQ ID NO:14)
**R**XC**P**XXXXXCXXXXXCXXXCXCXXXXXCX (SEQ ID NO:15)
**R**XCX**R**XXXXCXXXXXCXXXCXCXXXXXCX (SEQ ID NO:16)
X**V**C**P**XXXXXCXXXXXCXXXCXCXXXXXCX (SEQ ID NO:17)
X**V**CX**R**XXXXCXXXXXCXXXCXCXXXXXCX (SEQ ID NO:18)
XXC**PR**XXXXCXXXXXCXXXCXCXXXXXCX (SEQ ID NO:19)
**R**XCX**R**XXXXCXXXXXCXXXCXCXXXXXCX (SEQ ID NO:20)
**RV**C**P**XXXXXCXXXXXCXXXCXCXXXXXCX (SEQ ID NO:21)
**RV**CX**R**XXXXCXXXXXCXXXCXCXXXXXCX (SEQ ID NO:22)

XVCPRXXXXCXXXXXCXXXCXCXXXXXCX (SEQ ID NO:23)
RXCPRXXXXCXXXXXCXXXCXCXXXXXCX (SEQ ID NO:24)
RVCPRXXXXCXXXXXCXXXCXCXXXXXCX, (SEQ ID NO:25),

wherein in each of SEQ ID NO: 10-25,
X at position 9 is E or K, preferably K and X at position 21 is I or V, preferably V, and preferably wherein each of SEQ ID NO: 10-25 has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity with SEQ ID NO:1. In this paragraph, it is to be understood that R, V or P as identified in SEQ ID NO: 10-25 have the same meaning as in SEQ ID NO:9: R may be R or an analog of R, V may be V or an analog of V and P may be P or an analog of P.

The person skilled in the art will comprehend that conservative substitutions of amino acids can be made without changing the functional features of said peptide. Such conservative substitutions are within the scope of the present disclosure as long as the peptide remains a thermostable peptide which inhibits contact activation of the blood clotting system. Thermostability is preferably as defined earlier herein.

According to the invention, the thermostable peptide which inhibits contact activation of the blood clotting system, preferably comprises a peptide selected from the group consisting of:

- a peptide with the sequence as shown in SEQ ID NO: 1,
- a peptide with the sequence as shown in SEQ ID NO: 2,
- a peptide with the sequence as shown in SEQ ID NO: 3,
- a peptide with the sequence as shown in SEQ ID NO: 4, and
- a peptide which has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity with a peptide as shown in SEQ ID NO: 1, SEQ ID NO: 2, SEQ ID NO: 3, or SEQ ID NO: 4.

More preferably, the peptide comprises a peptide with the sequence as shown in SEQ ID NO: 1 or SEQ ID NO: 2. Even more preferably, the peptide comprises a peptide with the sequence as shown in SEQ ID NO:1.

According to the invention, the thermostable peptide which inhibits contact activation of the blood clotting system, preferably is a peptide selected from the group consisting of:

- a peptide with the sequence as shown in SEQ ID NO: 1,
- a peptide with the sequence as shown in SEQ ID NO: 2,
- a peptide with the sequence as shown in SEQ ID NO: 3,
- a peptide with the sequence as shown in SEQ ID NO: 4, and
- a peptide which has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity with a peptide as shown in SEQ ID NO: 1, SEQ ID NO: 2, SEQ ID NO: 3, or SEQ ID NO: 4.

More preferably, the peptide is a peptide with the sequence as shown in SEQ ID NO: 1 or SEQ ID NO: 2. Even more preferably, the peptide is a peptide with the sequence as shown in SEQ ID NO:1.

The person skilled in the art will comprehend that conservative substitutions of amino acids can be made without changing the functional features of said peptide.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value (of 10) more or less 0.1% of the value.

The sequence information as provided herein should not be so narrowly construed as to require inclusion of erroneously identified amino acids. The skilled person is capable of identifying such erroneously identified amino acids and knows how to correct for such errors.

**FIGURE LEGENDS**

**Figure 1:** Effect of the natural TICA-1 on thrombin generation triggered by tissue factor and by contact activation.

Drawn lines: No TICA1; dotted lines: 7.5 μg/ml TICA1. From left to right: Tissue factor triggered thrombin generation (2 overlapping peaks); contact activated thrombin generation (no TICA1); contact activated thrombin (with TICA1).

**Figure 2:** Contact activated thrombin generation, inhibition by CTI and TICA1 Drawn lines depict TICA-1. Concentrations from left to right: none, 1, 2, 3 and 5μM. Dotted line depicts CTI 0.75 μM.

**Figure 3:** Quantification of the inhibitory effects on factor XIIa. Circles represent data points; line represents best fit.

3a: effect of TICA1 (PT1) on hydrolysis of S2302 by h-FXIIa; FXIIa= 5nM; Kcal= $25s^{-1}$; Km= 180μM; Ki= 251nM
3b: effect of CTI on hydrolysis of S2302 by h-FXIIa; FXIIa= 4.21nM; Kcat= $25s^{-1}$; Km= 180μM; Ki= 2.90nM

**Figure 4:** Oxidative folding of TICA-1.

4a: HPLC of reduced (thick line) and oxidized (thin line) TICA-1 showing a typical reduction of retention time as a result of protein folding.
4b: Folded TICA-1; 4c: Unfolded TICA-1. Folding of TICA-1 results in a mass decrease of ∼ 6 Da, corresponding to the loss of 6 protons due to the formation of 3 disulfide bonds.

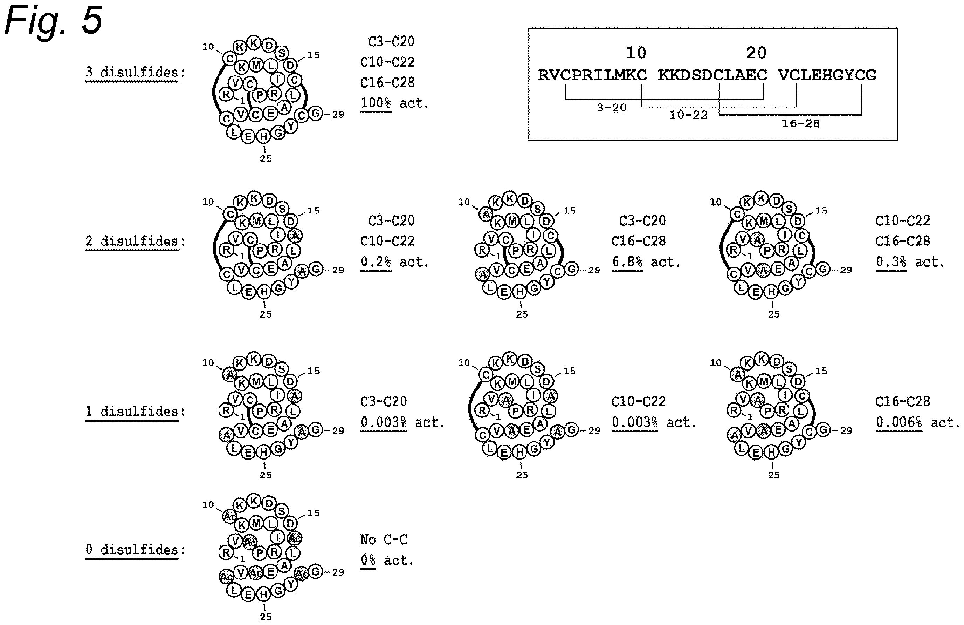

**Figure 5:** Thermostable Inhibitor of Coagulation Activation (TICA1): Dependence on Disulfides.
3 disulfides (C3-C20, C10-C22, C16-C28): 100 % activity
2 disulfides from 0.2 to 6.8 % activity:

(C3-C20, C10-C22): 0.2% activity
(C10-C22, C16-C28): 0.3 % activity
(C3-C20, C16-C28): 6.8 % activity
1 disulfide from 0.003 to 0.006% activity
(C10-C22): 0.003% activity
(C3-C20): 0.003% activity
(C16-C28): 0.006 % activity
No disulfide, no C: 0 activity

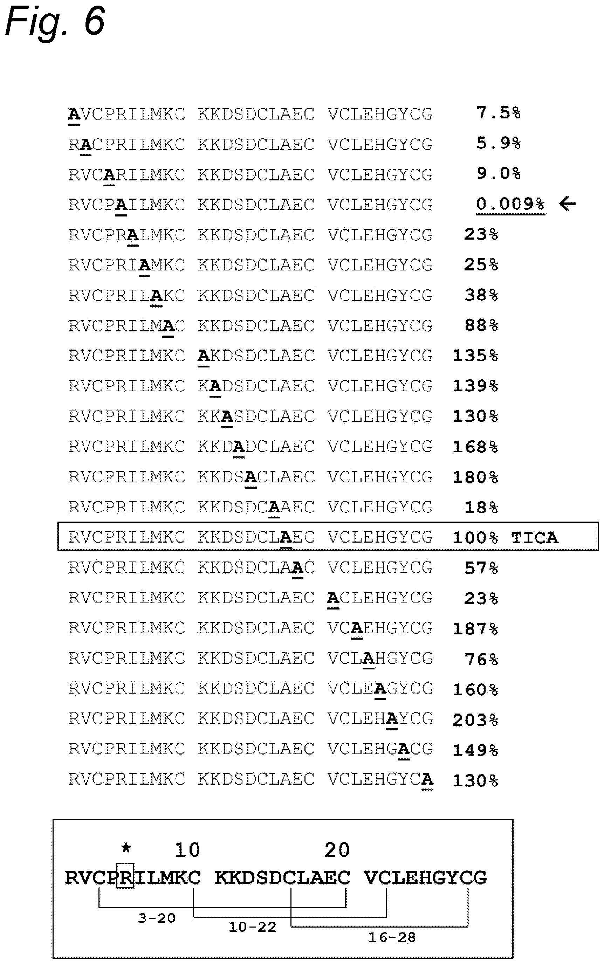

**Figure 6:** TICA1: Ala-scan with intact disulfide bonds.

## SEQUENCES

Sequences as set forth in the Sequence Listing

| SEQ ID NO: | ID | Gene product |
| --- | --- | --- |
| 1 | TICA 1 | RVCPRILMKCKKDSDCLAECVCLEHGYCG |
| 2 | TICA 2 | RVCPRILMECKKDSDCLAECVCLEHGYCG |
| 3 | TICA 3 | HEERVCPRILMKCKKDSDCLAECVCLEHGYCG |
| 4 | TICA 4 | RVCPKILMECKKDSDCLAECICLEHGYCG |
| 5 | TICA 5 | HEERVCPKILMECKKDSDCLAECICLEHGYCG |
| 6 | Consensus short | XILMXCKKDSDCLAECX |
| 7 | Consensus long | RVCPXILMXCKKDSDCLAECXCLEHGYCG |
| 8 | PCTI | RVCGIGPRPRLPWPRILMKCKKDSDCLAECVCLEHGYCG |
| 9 | Consensus | XXCXXXXXCXXXXXCXXXCXCXXXXXCX |

Wherein in SEQ ID NO: 6, X at position 1 is K or R, preferably R; X at position 5 is E or K, preferably K and X at position 17 is I or V, preferably V.
Wherein in SEQ ID NO: 7, X at position 5 is K or R, preferably R; X at position 9 is E or K, preferably K and X at position 21 is I or V, preferably V.

Wherein in SEQ ID NO: 9, X at position 5 is R or an analog of R,; X at position 9 is E or K, preferably K and X at position 21 is I or V, preferably V.

The present invention is further described by the following examples which should not be construed as limiting the scope of the invention.

Unless stated otherwise, the practice of the invention will employ standard conventional methods of molecular biology, virology, microbiology or biochemistry. Such techniques e.g. are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); "Laboratory techniques in thrombosis" Edited by J.Jespersen, R.M.Bertina and F.Haverkate , ISBN 0-7923-5317-X (1999); "Haemostasis and thrombosis: Basic principles and clinical practice" Eds: Colman, Hirsh, Marder, Clowes, George Lippincott Williams and Wilkins. (2010).

## EXAMPLES

### Summary

We isolated the trypsin inhibitors (serpins) from a multitude of seeds by affinity chromatography on a trypsin column and further purification procedures known to the art, notably the serpin from Cucurbita Maxima seeds, i.e. squash trypsin inhibitor (STI) (see example 2).

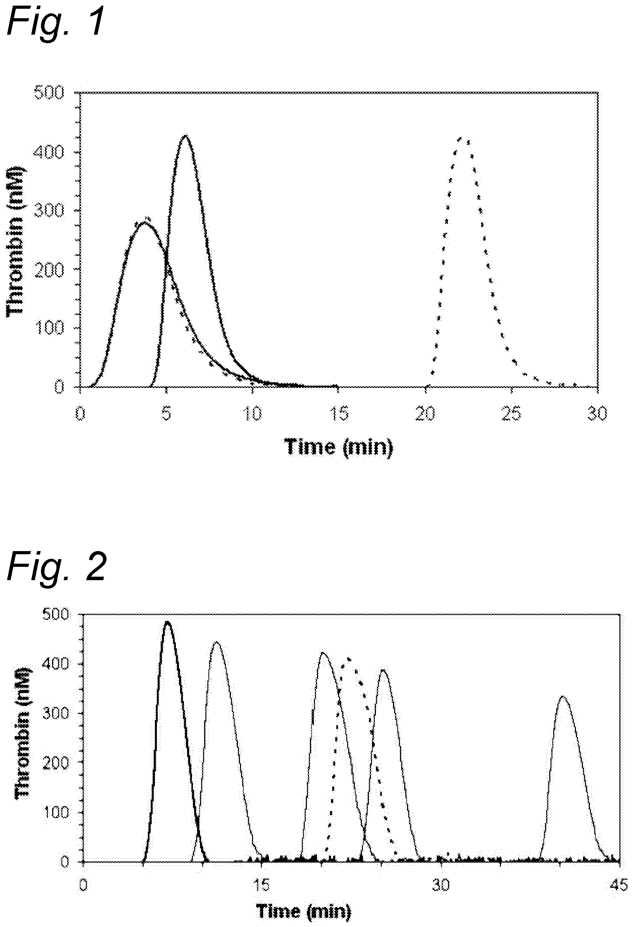

We confirmed that the serpin is an inhibitor of factor XIIa (see example 3). We observed that it effectively inhibited thrombin generation that was initiated by contact activation but not TG that was started by the natural trigger tissue factor (see figure 1). It should be noted here that inhibition of contact activation is seen in thrombin generation curves as a prolongation of the lag time of thrombin generation because contact activation produces factor IXa and a certain critical amount of factor IXa is required to start thrombin generation.

That tissue factor driven thrombin generation is not inhibited indicates that STI does not inhibit the natural clotting process but only contact activation. Indeed STI inhibited contact activation so well that blood taken on a solution not containing sodium citrate or another substance that binds $Ca^{2+}$ ions but only STI (10 $\mu$g/ml) remained fluid for over 45 minutes, under conditions where in absence of STI it would clot in less than 5 min. If a tube containing STI was sterilized by heating for one hour in boiling water the STI, surprisingly, did not loose its inhibitory activity: blood would still clot after > 45 min only. CTI under similar circumstances, at 10 $\mu$g/ml, lost much of its activity because a clotting time of < 15min. was observed.

The natural product was further analysed and found to contain two active polypeptides, which were purified to homogeneity (example 2).

These two natural peptides (TICA1 and TICA2) were further analysed to determine their primary structure by means of Tandem Matrix-assisted Laser Desorption Ionisation Time-of-flight (MALDI-TOF) mass spectrometry in conjunction with peptide mass fingerprinting (PMF) of fragments obtained by trypsinolysis. PMF positive fragments were confirmed by collision-induced fragmentation (CID) and amino acid sequence determination. The primary structures found is shown in table 1.

**Table 1.** *Aminoacid sequence and Molecular Weight(MW) of TICA 1-5*

| SEQ ID NO: | ID | Gene product | MW Reduced | MW folded |
|---|---|---|---|---|
| 1 | TICA 1 | RVCPRILMKCKKDSDCLAECVCLEHGYCG | 3274 | 3268 |
| 2 | TICA 2 | RVCPRILMECKKDSDCLAECVCLEHGYCG | 3275 | 3269 |
| 3 | TICA 3 | HEERVCPRILMKCKKDSDCLAECVCLEHGYCG | 3669 | 3663 |
| 4 | TICA 4 | RVCPKILMECKKDSDCLAECICLEHGYCG | 3260 | 3254 |
| 5 | TICA 5 | HEERVCPKILMECKKDSDCLAECICLEHGYCG | 3656 | 3650 |

TICA 1 and 2 we then synthesized by means of Solid Phase Peptide Synthesis together with several congeners (Table 1, TICA 3, 4 and 5)(see example 4).

We determined the inhibitory action of the synthetic materials in the same way as we had done for the natural ones and found TICA1 to be the most active (see table 2 and example 3).

Table 2 Inhibitory activity of different polypeptides

| Inhibitor | CTI | STI | TICA1 | TICA2 | TICA3 | TICA4 | TICA5 |
|---|---|---|---|---|---|---|---|
| Ki (nM) | 2.9 | 251 | 25.5 | 109 | 3090 | 1420 | 171000 |

We then determined the action of TICA1 as an inhibitor of contact activation. It appeared to prolong the lag time of thrombin generation that was triggered by contact activation, in the same manner as STI did (see figure 1).

TICA1 was further modified by inserting the CTI sequence around the reactive bond, PCTI (SEQ ID NO: 8). The resulting PCTI peptide was inactive indicating that the new found inhibitors have no structure-function relationship in common with CTI. The effect of TICA1 on contact activation of PPP, elicited by incubation with kaolin, was compared with the effect of CTI. As can be seen in table 3 and figure 2, there is a dose-dependent inhibition of contact activation when PPP is incubated with various concentrations of TICA1 (0.94 to 4.6 $\mu$M). On a molar basis, peptide B is ∼ 2.5 times less potent than CTI is. On a weight basis it is equipotent.

Table 3 Influence of TICA1 on contact activation.

| Added | $\mu$M | $\mu$g/ml | Lag-time | ETP | Peak | ttPeak |
|---|---|---|---|---|---|---|
| TF | | | 1.5 | 1250 | 374 | 3 |
| Kaolin | | | 4.81 | 1209 | 454 | 6.06 |
| Kaolin-CTI | 0.72 | 8.64 | 18.62 | 1116 | 385 | 20.06 |
| Kaolin-PTI | 0.94 | 3.76 | 9.81 | 1146 | 419 | 11.06 |
| Kaolin-PTI | 1.87 | 7.48 | 17.69 | 1144 | 405 | 18.94 |
| Kaolin-PTI | 2.81 | 11.24 | 23.88 | 1092 | 375 | 25.38 |
| Kaolin-PTI | 3.74 | 14.96 | 19.69 | 1070 | 355 | 21.25 |
| Kaolin-PTI | 4.68 | 18.72 | 38.69 | 949 | 324 | 40.19 |

TICA1 has a MW = 3268 Da and is easy to synthesize, fold and purify.
It can be left for weeks at room temperature without loosing activity. Heating a 0.1 mM solution in a closed ampoule in boiling water for 60 min does not affect its biological activity whereas CTI is significantly inactivated. (Example 7) This means that, unlike CTI, it can be added to citrate tubes that can be sterilized and used under clinical conditions. About 0.10 mg of TICA1 would be required per vacuum tube meant to take 10 ml of blood.

We thus provided a class of polypeptides, the TICAs (Thermostable Inhibitors of Contact Activation) that are specific inhibitors of the contact activation system of blood coagulation and therefore can be profitably used to prevent this system from disturbing the function analysis of thrombin generation in blood samples in the same manner as the previously patented corn trypsin inhibitor, with the additional advantage of heat stability and hence the possibility of sterilisation which greatly enhances its use in routine medical diagnostic practice.

## Example 1

Thrombin generation in presence of the various polypeptides.

Thrombin generation was carried out according to WO2003/093831.

Venous blood was collected into tubes containing 0.106 mol/l tri-sodium citrate (1:9, v:v). from healthy adult volunteers after obtaining informed consent. Following a double centrifugation at 2500xg for 15 min at room temperature, platelet poor plasma (PPP) was collected from the upper half volume of plasma supernatant, quick frozen and stored at -80°C. The absence of platelet and leucocyte in PPP was checked with an ADVIA 120 counter (Bayer Diagnostics, NY, USA).

## Reagents for thrombin generation test

Recombinant human tissue factor Innovin® was obtained from Dade Behring (Marburg, Germany) and used at a final concentration of 0.5 pM in PRP and 1 or 5 pM in PPP samples. The phospholipid vesicles used at a final concentration of 4 $\mu$M, were obtained from Avanti Polar Lipids (Alabaster, Alabama, USA) and consisted of 20 mol% phosphatidylserine (PS), 20 mol% phosphatidylethanolamine (PE) and 60 mol% phosphatidylcholine (PC) and were prepared by extrusion method (9,10). Kaolin light (hydrated aluminium silicate) was obtained from Baker Harrison ltd, (Ilford, UK). Hepes-buffered saline contained 20mM Hepes (Sigma Aldrich, l'Ile d'Abeau Chesnes, France), 140mM NaCl and 5mg/mL bovine serum albumin (BSA) (Euromedex, Souffelweyersheim, France), pH 7.35. This buffer was stored at -20°C until use. A fresh mixture of fluorogenic substrate and $CaCl_2$ was prepared before each experiment. Fluorogenic substrate, Z-Gly-Gly-Arg-AMC, was obtained from Bachem (Bubendorf, Switzerland). The mixture of fluorogenic substrate 2.5 mM and $CaCl_2$ 0.1 M was prepared using buffer containing Hepes 20mM and 60mg/mL BSA, pH 7.35. The Calibrator with the activity of 600 nM human thrombin was obtained from Thrombinoscope BV (Maastricht, The Netherlands). Transparent, round bottom Greiner microtiter plates (Greiner ref 65204, Poitiers, France) were used.

**Calibrated Automated Measurement of Thrombin Generation (CAT)**

Thrombin generation was measured using Calibrated Automated Thrombography (CAT) according to the methodology of WO2003/093831 and a 96-well plate fluorometer (Fluoroscan Ascent Reader, Thermolab systems OY, Helsinki, Finland) equipped with a 390/460nm filter set. Briefly, 80$\mu$L of plasma is dispensed into the wells of round-bottom 96 well-microtiter plates. 20$\mu$L of a mixture containing tissue factor and phospholipids is added to the PPP sample; alternatively, in order to obtain contact activation, the citrated plasma is preincubated for at least 10 min with 1 mg/ml of Kaolin light. The starting reagent (20$\mu$L per well) contains fluorogenic substrate and $CaCl_2$. A dedicated software program, Thrombinoscope® (Thrombinoscope bv, Maastricht, The Netherlands) enabled the calculation of thrombin activity against the calibrator (Thrombinoscope bv, Maastricht, The Netherlands) and displayed thrombin activity with the time. The most important parameters that can be derived from CAT are lag time, endogenous thrombin potential corresponding to the area under the CAT curve, peak height of thrombin and time to peak.

**Example 2**

*Purification of the serpin STI from Curbita Maxima seeds.*

STI is purified from *Cucurbita maxima* seeds in a three step procedure:

1: Extraction of crushed seeds with 0.1 M TRIS buffer, pH 8.0 and
2: Affinity chromatography on a Trypsin-Sepharose column
3: Reversed Phase (RP) HPLC - C18 column, linear gradient 0-60% acetonitrile in water 0.1% trifluoroacetic acid

This allows the separation of two active peptides (TICA1 and TICA2) of which the amino-acid sequence was determined (see Table 1).

**Example 3**

*Inhibition of factor XIIa by serpins*

*Materials*

S2302 From Chromogenic, lot # N0398718 SEQ0860, exp. date 2012-07. Water added on 7.10.2010 → 3.7 mM. On 3.08.10 the OD at 316 nm was measured (0.236+0.235) / 2 $\times$ 200 = 47.1. The calculated concentration thus is 47.1 / 12.9 = 3.7 mM. The kinetics of S2302 hydrolysis by FXIIa was $K_m$ = 180 $\mu$M and $k_{cat}$ = 25 s$^{-1}$.

h-FXIIa From "Enzyme Research Laboratories". Product code FXIIa 1212A, lot # FXIIa 2520PL. Bottle with 0.5 mg was reconstituted with 370 $\mu$l pure water. Protein concentration: 1.35 mg/ml, i.e. ~8 $\mu$M. Buffer 4 mM NaAc, 150 mM NaCl (pH 5.3). Preparation was divided into 20 $\mu$l amounts and frozen at -80 °C.

Hepes735 140 mM NaCl, 20 mM Hepes, 0.02% $NaN_3$ (pH 7.35).

BSA5 Hepes735 + 5 mg/ml BSA.

CTI Prepared on 17.12.2004, 0.141 mg/ml, i.e. 0.141/12028 = 11.7*10$^{-6}$ M (11.7 $\mu$M). One portion was thawed and divided into 3 parts. Part 1 was frozen at -80 °C; part 2 was kept at RT; and part 3 was incubated 30 min at 95°C and then kept at RT.

*Effect of CTI, STI and TICA1 on hydrolysis of S2302 by h-FXIIa*

Stock FXIIa was diluted 700 $\times$ in BSA5. The inhibitor was diluted in BSA5 as indicated. Wells contained 70 $\mu$l diluted FXIIa (5 nM final), 70 $\mu$l inhibitor and 20 $\mu$l S2302 (475 or 462.5 $\mu$M final). The reaction was started after 5 min heating on the heating plate with S2302. Filters 405 nm and 490 nm reference. Found m$\Delta$A/min was multiplied by 2.14 to get the figure for a 1 cm path.

The residual activity (V) was fitted against the PTI concentration with equation:

$$V = (E+(sqrt(E\char`^2+2*E*(Ki-I)+I\char`^2+2*I*Ki+Ki\char`^2)-E-I-Ki)/2)*kcat*S/(Km+S)$$

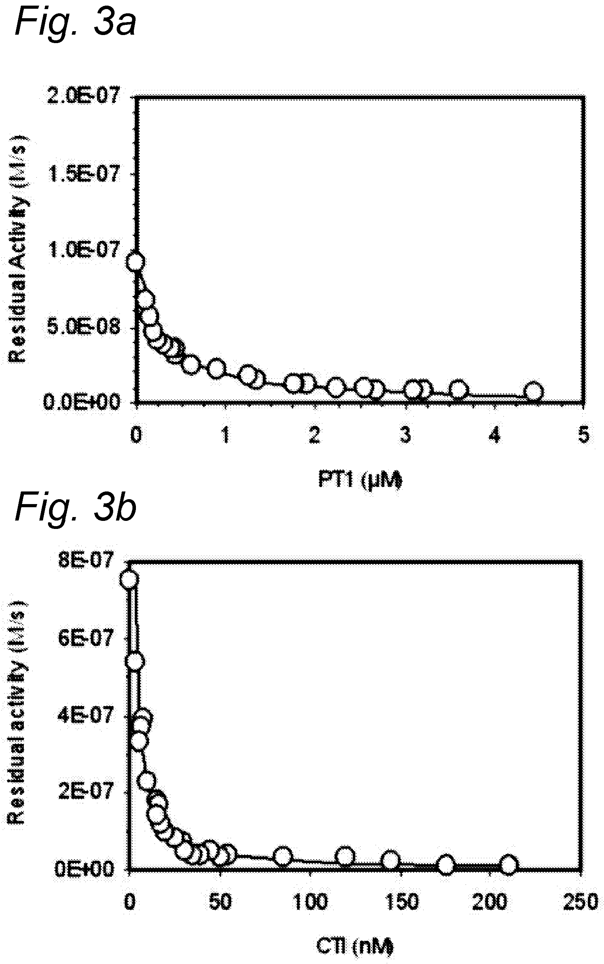

in which E = [FXIIa], Ki is the dissociation constant, I = [Inhibitor], kcat and Km are Michaelis constants and S = [S2302]. Figure 3 shows these fits and the derived constants for PTI and CTI. Table 4 summarizes the constants thus found.

**Table 4** Dissociation constants of h-FXIIa - inhibitor complexes.

| Inhibitor | Dissociation constant (Ki) (nM) |
|---|---|
| CTI | 2.9 |
| STI | 251 |
| TICA1 | 25.5 |
| TICA2 | 109 |
| TICA3 (= HEE-TICA1) | 3,090 |
| TICA4 | 1,420 |
| TICA5 (= HEE-TICA4) | 171,000 |
| AAA-TICA1 | 104 |
| TICA1-AAA | 377 |
| PCTI | 469,000 |

### Example 4

*Chemical synthesis of TICA1*

General: 1.TICA1 was synthesised by solid phase peptide synthesis, cleaved from the resin (solid phase) and simultaneously deprotected, purified by HPLC, and lyophilized. 2. Purified TICA1 was dissolved, and oxidatively folded to obtain the disulfide bonded three-dimensional active structure. Final product was purifed by HPLC and lyophilised.

Specifically:

1. Synthesis: TICA1 polypeptide chain was synthesized by manual solid-phase peptide synthesis on a 0.2 mmol scale using the in situ neutralization/activation procedure for Boc-/Bzl- peptide synthesis as previously described, 10 but using HCTU instead of 2-(1H-benzotriazole-1-yl)-1,1,3,3-tetramethyluronium hexafluorophosphate (HBTU) as a coupling reagent. Glycyl PAM resin (0.76 meq/g) was used as the solid support. TICA1 peptide was deprotected and cleaved from the resin by treatment with anhydrous HF for 1 h at 0 °C, using 4 v-% p-cresol as a scavenger. Following cleavage, TICA1 polypeptide chain was precipitated with ice-cold diethylether, dissolved in aqueous buffer containing 6 M Gn.HCl, 0.1 M sodium acetate buffer (pH 4) and purified by semi-preparative HPLC. Fractions containing the desired product were identified by ESI-MS, pooled and lyophilized.

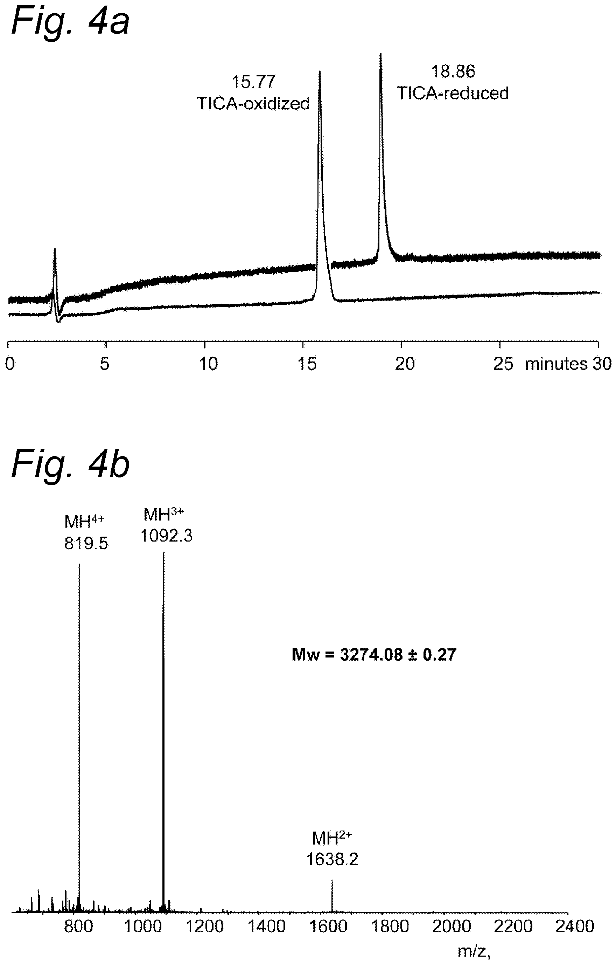

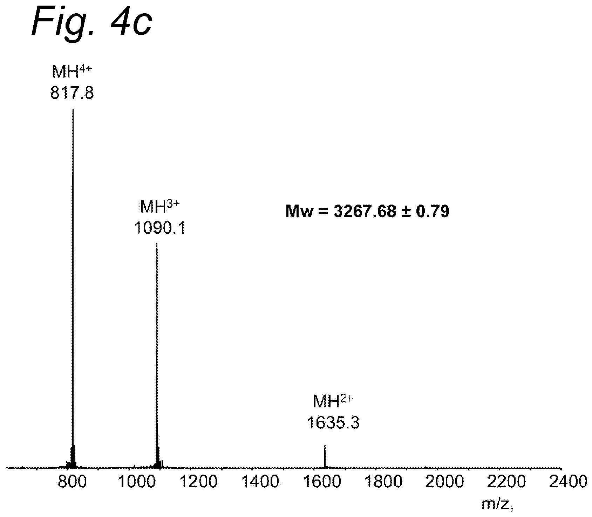

2. Oxidative Protein Folding: TICA1 (H-Arg1-Gly29-OH) (18 mg, 5.49 μmole) was dissolved in 50 mL of 1 M Gn.HCl, 0.10 M Tris buffer (pH 8.0) containing 8 mM cysteine and 1 mM cystine. The resulting solution was stirred for 2h at 4 °C. Reaction progress was monitored by analytical HPLC (Fig. 4). After semi-preparative HPLC, product containing fractions were identified by ESI-MS, pooled and lyophilized to give 10.05 mg of oxidized TICA1 (3.08 μmole, 56 %). ESI-MS showed a mass of 3267.68 +/- 0.79, fitting well between the calculated monoisotopic mass (3266.50) and average mass (3268.99 of the folded TICA1. The observed mass difference of 6.4 Da between unfolded and folded TICA1 corresponds to the expected mass reduction caused by the loss of 6 protons due to the formation of 3 disulfide bonds (Fig. 4).

## Example 5

*Heat stability of the inhibitory products.*

The following table shows the inhibitory activity of some of these products on the small molecular weight chromogenic peptide S2302, i.e. H-D-Pro-Phe-Arg-p.nitroaniline before and after heating, as well as the concentration that prolongs the clotting time of plasma that is optimally contact activated by preincubation with kaolin four times.

**Table 5** Heat stability of inhibitory products.

| Inhibitor | Heating | TICA2 | TICA1 | TICA3 | TICA4 | TICA5 |
|---|---|---|---|---|---|---|
| | | | | | | |
| $k_i$ F.XIIa (ng/ml) | Before | 355 | 83 | 10095 | 4621 | 556434 |
| $k_i$ F.XIIa (ng/ml) | After | | 85 | | | |
| $k_i$ F.XIIa (nM) | Before | 109 | 25.5 | 3090 | 1420 | 171000 |
| $k_i$ F .XIIa (nM) | After | | 26.1 | | | |
| 4 x lag-time ($\mu$M) | Before | 9.6 | 2.4 | ND | 31.6 | ND |
| 4 x lag-time ($\mu$M) | After | 9.8 | 2.3 | ND | 31.4 | ND |
| 4 x lag-time (ng/ml) | Before | 31.1 | 7.8 | ND | 102.7 | ND |
| 4 x lag-time (ng/ml) | After | 31.9 | 7.5 | ND | 102.9 | ND |

## Example 6

*Stability of CTI, PTI, and TICA at RT and 30 min at 95 °C*

Stock FXIIa was diluted 700 $\times$ in BSA5. The inhibitor was diluted in BSA5 as indicated. Wells contained 70 $\mu$l diluted FXIIa (5 nM final), 70 $\mu$l inhibitor and 20 $\mu$l S2302 (475 or 462.5 $\mu$M final). The reaction was started after 5 min heating on the heating plate with S2302. Filters 405 nm and 490 nm reference. Found $\Delta$mA/min was multiplied by 2.14 to get the figure for a 1 cm path.
The residual activity (V) was fitted against the inhibitor concentration with equation:

$$V = (E+(sqrt(E^2+2*E*(Ki-I)+I^2+2*I*Ki+Ki^2)-E-I-Ki)/2)*kcat*S/(Km+S)$$

in which E = [FXIIa], Ki is the dissociation constant, I = [Inhibitor], kcat and Km are Michaelis constants and S = [S2302].

**Table 6** Effect of temperature of CTI, PTI and TICA on its inhibition of h-FXIIa (5.01 nM)

| Inhibitor | Condition | (Ki) (nM) | Res. Act. (%) |
|---|---|---|---|
| CTI | -80°C | 3.59 | 100 |
| | RT during 72 hrs | 4.19 | 86 |
| | 30 min 95°C | 5.10 | 70 |
| PTI | -80°C | 251 | 100 |
| | RT during 72 hrs | 267 | 106 |
| | 30 min 95°C | 264 | 105 |
| TICA1 | -80°C | 26 | 100 |
| | RT during 72 hrs | 26 | 102 |
| | 30 min 95°C | 24 | 94 |
| TICA2 | -80°C | 109 | 100 |

(continued)

| Inhibitor | Condition | (Ki) (nM) | Res. Act. (%) |
|---|---|---|---|
| | RT during 72 hrs | 114 | 105 |
| | 30 min 95°C | 108 | 99 |
| TICA3 | -80°C | 3090 | 100 |
| | RT during 72 hrs | 3128 | 101 |
| | 30 min 95°C | 3180 | 103 |
| TICA4 | -80°C | 1420 | 100 |
| | RT during 72 hrs | 1398 | 98 |
| | 30 min 95°C | 1337 | 94 |
| TICA5 | -80°C | 171000 | 100 |
| | RT during 72 hrs | 164531 | 96 |
| | 30 min 95°C | 179243 | 105 |

Exposure of CTI and TICA1 for 60 minutes to 95°C

To calculate the concentration of I the equation $I = x \cdot (x-E-Ki) / (x-E)$ was used. In this equation is I the calculated inhibitor concentration, x = the residual activity of FXIIa (free FXIIa), E the total FXIIa concentration (= 9.33 nM), and Ki the dissociation or inhibitor constant.

| Inhibitor | Added amount (nM) | Activity of FXIIa (mA/min) | Free FXIIa (nM) | Calculated total I (nM) | Residual activity (%) |
|---|---|---|---|---|---|
| No Inhibitor | - | $79.6 \pm 1.55$ | 9.33 | - | 100 |
| CTI | 8.1 | $66.3 \pm 1.32$ | 7.77 | 2.37 | 29.3 |
| TICA1 | 306 | $33.5 \pm 1.78$ | 3.93 | 250 | 81.7 |

## Example 7

*Stability of TICA1 in relation to amount of disulfide bonds (0-1-2-3)*

Chemical synthesis of TICA1 with 3 disulfide bonds is described in example 4

In order to synthesize TICA1 with less disulfide bonds, the synthesis and oxidative protein folding is different.

TICA1 *with 2 disulfide bonds*

Specifically:

1. Synthesis: TICA1 with 2 disulfide bonds was synthesized by manual solid-phase peptide synthesis as in example 4 except for the cystein residues. Two cystein residues responsible for a disulfide bond were replaced by alanine. Two other cystein residues responsible for a disulfide bond were replaced by cystein protected with acetamidomethyl (Acm) groups. The third cystein residues responsible for a disulfide bond were unchanged. The peptide was deprotected and cleaved as described in example 4. After HF cleavage, two cysteines are unprotected, the other two stayed protected with acetamidomethyl (Acm) groups. TICA1 polypeptide chain was precipitated with ice-cold diethylether, dissolved in aqueous buffer containing 50% acetonitril and 0.1% TFA, was analyzed by HPLC and lyophilized.

2. Oxidative Protein Folding: In order to generate the first disulfide bond, TICA1 was dissolved in 1 M Gn.HCl, 0.05 M Tris buffer (pH 8.0) (0.5mg/ml). The resulting solution was stirred for 48 h at 4 °C. Then for the formation of the second disulfide bond the solution was adjusted to 10% AcOH, purged with nitrogen, and the Acm groups were removed by addition of 2 equivalents Iodine (0.12M in methanol). Reaction progress was monitored by analytical HPLC and ESI-MS, showing a mass fitting well between the calculated monoisotopic mass and average mass of the folded peptide. After semi-preparative HPLC, product containing fractions were identified by ESI-MS, pooled and lyophilized. The observed mass difference of -144.2 Da corresponds to the expected mass reduction caused by the loss of 2 protons and 2 Acm groups due to the formation of 2 disulfide bonds.

TICA1 *with 1 disulfide bond*

Specifically:

1. Synthesis: TICA1 with 2 disulfide bonds was synthesized by manual solid-phase peptide synthesis as in example 4 exept for the cystein residues. Four cystein residues responsible for 2 disulfide bonds were replaced by alanine. The peptide was deprotected and cleaved as described in example 4. Following cleavage, TICA1 polypeptide chain was precipitated with ice-cold diethylether, dissolved in aqueous buffer containing 6 M Gn.HCl, 0.1 M sodium acetate buffer (pH 4) and purified by semi-preparative HPLC. Fractions containing the desired product were identified by ESI-MS, pooled and lyophilized.

2. Oxidative Protein Folding: Oxidative Protein Folding: TICA1 was dissolved in 1 M Gn.HCl, 0.10 M Tris buffer (pH 8.0) The resulting solution was stirred for 24h at 4 °C. Reaction progress was monitored by analytical HPLC (Fig. 4). After semi-preparative HPLC, product containing fractions were identified by ESI-MS, pooled and lyophilized. ESI-MS showed an observed mass difference of 2 Da between unfolded and folded TICA1 corresponds to the expected mass reduction caused by the loss of 2 protons due to the formation of 1 disulfide bond (Fig. 4).

TICA1 *without disulfide bonds*

Specifically:

1. Synthesis: TICA1 was synthesized by manual solid-phase peptide synthesis as in example 4. The peptide was deprotected and cleaved as described in example 4. Following cleavage, reduced TICA1 polypeptide chain was precipitated with ice-cold diethylether, dissolved in aqueous buffer containing 6 M Gn.HCl, 0.1 M sodium acetate buffer (pH 4) and purified by semi-preparative HPLC. Fractions containing the desired product were identified by ESI-MS, pooled and lyophilized.

2. Blocking Cysteines ("Ac" in example 7): reduced TICA1 was dissolved in 50mM ammonium bicarbonate pH 7.8 (1mg/ml). 40 mM Iodoacetamide (7.4 mg/ml) was added. The reaction took place for 1 hour in the dark. Reaction progress was monitored by analytical HPLC After semi-preparative HPLC, product containing fractions were identified by ESI-MS, pooled and lyophilized. ESI-MS showed an observed mass difference of 342 Da between unblocked and blocked cysteins corresponds to the expected mass increase caused by adding 6 acetamides to the cysteines.

### Example 8

*Ala-scan with intact Disulfides*

The contribution of every amino acid to the activity of TICA1 was determined. Every single amino acid was substituted by alanine except for cysteine. The effect of cysteine in TICA1 was already described in example 7. All TICA1-analoges were synthesized and folded as described in example 4. See also figure 6.

| Sequence Ala scan | Activity vs TICA1 |
|---|---|
| **A**VCPRILMKCKKDSDCLAECVCLEHGYCG | 7.5% |
| R**A**CPRILMKCKKDSDCLAECVCLEHGYCG | 5.9% |
| RVC**A**RILMKCKKDSDCLAECVCLEHGYCG | 9.0% |
| RVCP**A**ILMKCKKDSDCLAECVCLEHGYCG | 0.009% |
| RVCPR**A**LMKCKKDSDCLAECVCLEHGYCG | 23% |

(continued)

| Sequence Ala scan | Activity vs TICA1 |
|---|---|
| RVCPRI**A**MCKKDSDCLAECVCLEHGYCG | 25% |
| RVCPRIL**A**KCKKDSDCLAECVCLEHGYCG | 38% |
| RVCPRILM**A**CKKDSDCLAECVCLEHGYCG | 88% |
| RVCPRILMKC**A**KDSDCLAECVCLEHGYCG | 135% |
| RVCPRILMKCK**A**DSDCLAECVCLEHGYCG | 139% |
| RVCPRILMKCKK**A**SDCLAECVCLEHGYCG | 130% |
| RVCPRILMKCKKD**A**DCLAECVCLEHGYCG | 168% |
| RVCPRILMKCKKDS**A**CLAECVCLEHGYCG | 180% |
| RVCPRILMKCKKDSDC**A**AECVCLEHGYCG | 18% |
| RVCPRILMKCKKDSDCL**A**ECVCLEHGYCG | 100% (=TICA1) |
| RVCPRILMKCKKDSDCLA**A**CVCLEHGYCG | 57% |
| RVCPRILMKCKKDSDCLAEC**A**CLEHGYCG | 23% |
| RVCPRILMKCKKDSDCLAECVC**A**EHGYCG | 187% |
| RVCPRILMKCKKDSDCLAECVCL**A**HGYCG | 76% |
| RVCPRILMKCKKDSDCLAECVCLE**A**GYCG | 160% |
| RVCPRILMKCKKDSDCLAECVCLEH**A**YCG | 203% |
| RVCPRILMKCKKDSDCLAECVCLEHG**A**CG | 149% |
| RVCPRILMKCKKDSDCLAECVCLEHGYC**A** | 130% |

**Example 9**

*The effect of TICA addition to blood collection tubes and CAT assay*

In 12 donors blood was drawn with 7 different drawing tubes with and without addition of TICA1 in the drawing tubes (total 14 tubes; 78 ml). After preparing PPP from these tubes, CAT measurements with MP reagent and pplow reagent was performed. One donor is measured in one plate. (pplow duplicate; MP duplicate; calibrator in 4 fold. Total volume is 320 μl sample/measurement in total 88 wells/donor.

In 12 donors blood is taken with the following commercial available drawing tubes:
(From all blooddrawing tubes one tube with and one tube without TICA will be used to draw blood)
Start blooddrawing with one edta tube.

1A: BD vacutainer (glass) 9NC; 0.5 ml 0.105M citrate, total volume 5 ml.

1C: Add to one vacuum tube: 100 μl, 1.5 mg/ml TICA
Total tubes: 2 => one tube with TICA and one tube without TICA.

2A: Sarstedt tubes; S-monovette, 9NC; 0.5 ml 0.106M citrate, total volume 5 ml.

2C: Add to one vacuum tube: 100 μl, 1.5 mg/ml TICA
Total tubes: 2 => one tube with TICA and one tube without TICA.

3A: Terumo Venosafe 4 ml. 9NC; 0.4 ml 0.109M citrate, total volume 4 ml. ppg internal coating / silicone stopper coating

3C: Add to one vacuum tube: 80 μl, 1.5 mg/ml TICA.
Total tubes: 2 => one tube with TICA and one tube without TICA.

4A: BD Vacutainer, (plastic) 9NC; 0.3 ml 0.109M citrate, total volume 3 ml.

4C: Add to one vacuum tube: 60 μl, 1.5 mg/ml TICA.
Total tubes: 2 => one tube with TICA and one tube without TICA.

5A: Haemtech SCAT 113-4.5/5 AA0809 3.2% citrate.(Control tubes without CTI), total volume 5 ml.

5C: Add to one vacuum tube: 100 µl, 1.5 mg/ml TICA.
Total tubes: 2 => one tube with TICA and one tube without TICA.

6A: Greiner vacuum tubes 9 ml + 1 ml (9NC, 3.2% citrate)

6C: Add to one vacuum tube: 200 µl, 1.5 mg/ml TICA.
Total tubes: 2 => one tube with TICA and one tube without TICA.

Final TICA concentration after blood drawing in every tube with added TICA is 30 µg/ml whole blood. Final TICA concentration in plasma after preparing PPP is 60 µg/ml (assuming an hematocit of 50%)

TICA preparation:

Dissolve 9 mg TICA in 6 ml sterile injection water. This solution is used for injection of TICA in the drawing tube. After injection of TICA solution in the blood drawing tube this tube has to be used within 8 hours.

CAT

Measurement no TICA and TICA added to Tube:

CAT measurements with MP reagent and pplow reagent will be performed. One donor is measured in one plate. PPLow duplicate; MP duplicate; calibrator in 4 fold.
Total volume is 320 µl sample/measurement in total 88 wells/donor. NP2012 is also included in the measurement in 4 fold.
Lag times, Endogenous thrombin potentials, and peak heigth were recorded, For the current conditions of thrombin generation, peak height is the preferred parameter for interpretation of the results.

Measurement TICA added to CAT assay:

Plasma with TICA added: concentration in plasma: 60 µg/ml

Stock TICA: 1.5 mg/ml => 1500 µg/ml

20 µl TICA (stock: 1500µg/ml) is added to 480 µl plasma.

| TICA series in 9 volunteers | No Tica | TICA added to CAT assay | TICA added to Tube |
|---|---|---|---|
| BD Glass 0 pM TF Lagtime (min) | 12.8 ± 3.2 | 38.8 ± 34.8 | 76.6 ± 34.0 |
| BD Glass 0 pM TF ETP (nM IIa.min) | 1053 ± 334 | 760 ± 526 | 77 ± 119 |
| BD Glass 0 pM TF Peak Height (nM IIa) | **165 ± 61** | **105 ± 81** | **6 ± 9** |
| BD Plastic 0 pM TF Lagtime (min) | 14.8 ± 6.5 | 49.5 ± 37.7 | 90.1 ± 26.8 |
| BD Plastic 0 pM TF ETP (nM IIa.min) | 910 ± 472 | 614 ± 538 | 20 ± 61 |
| BD Plastic 0 pM TF Peak Height (nM IIa) | **162 ± 71** | **86 ± 87** | **2 ± 6** |
| Sarstedt 0 pM TF Lagtime (min) | 16.7 ± 16.9 | 50.0 ± 38.0 | 52.5 ± 36.2 |
| Sarstedt 0 pM TF ETP (nM IIa.min) | 921 ± 445 | 279 ± 227 | 140 ± 106 |
| Sarstedt 0 pM TF Peak Height (nM IIa) | **126 ± 70** | **26 ± 23** | **10 ± 8** |
| Terumo 0 pM TF Lagtime (min) | 26.1 ± 29.9 | 64 ± 40.6 | 75.7 ± 35.6 |
| Terumo 0 pM TF ETP (nM IIa.min) | 846 ± 523 | 276 ± 367 | 59 ± 119 |
| Terumo 0 pM TF Peak Height (nM IIa) | **117 ± 95** | **33 ± 50** | **5 ± 11** |
| Greiner 0 pM TF Lagtime (min) | 13.1 ± 3.0 | 31.2 ± 26.7 | 65.0 ± 34.8 |
| Greiner 0 pM TF ETP (nM IIa.min) | 992 ± 313 | 834 ± 449 | 144 ± 164 |
| Greiner 0 pM TF Peak Height (nM IIa) | **147 ± 62** | **110 ± 70** | **11 ± 13** |
| Heamtech 0 pM TF Lagtime (min) | 21.7 ± 29.2 | 46.4 ± 39.7 | 65.0 ± 34.8 |
| Heamtech 0 pM TF ETP (nM IIa.min) | 981 ± 521 | 666 ± 552 | 289 ± 420 |

(continued)

| TICA series in 9 volunteers | No Tica | TICA added to CAT assay | TICA added to Tube |
|---|---|---|---|
| Heamtech 0 pM TF Peak Height (nM IIa) | 155 ± 90 | 93 ± 85 | 36 ± 62 |

REFERENCE LIST

1. US6,403,381.
2. WO2010/016762
3. WO2003/093831
4. WO2006/117246
5. "The blood coagulation cascade" by Schenone M, Furie BC and Furie B. Current Opinion in Haematology 2004, Vol 11(4) pages 272-277.
6. L.Poller in "Laboratory techniques in thrombosis" Edited by J.Jespersen, R.M.Bertina and F.Haverkate , ISBN 0-7923-5317-X (1999) p.37-44.
7. "Laboratory techniques in thrombosis" Edited by J.Jespersen, R.M.Bertina and F.Haverkate , ISBN 0-7923-5317-X (1999);
8. "Haemostasis and thrombosis: Basic principles and clinical practice" Eds:Colman, Hirsh, Marder, Clowes, George Lippincott Williams and Wilkins. (2010).

**Claims**

1. A composition comprising at least one calcium-chelating substance with blood clotting inhibition activity and further comprising a thermostable peptide of at most 42 amino acids which inhibits contact activation of the blood clotting system, said peptide comprising the consensus amino acid sequence: RVCPXILMXCKKDSDCLAECXCLEHGYCG (SEQ ID NO: 7), wherein X at position 5 is K or R or an analog of R, preferably R or an analog of R; X at position 9 is E or K, preferably K; and X at position 21 is I or V, preferably V , wherein an analog of Arg is selected from the group consisting of: monomethylarginine, symmetric and asymmetric dimethylarginine, canaline, δ-guanidino α-amino butyric acid, homoarginine, canavanine, and citrulline, and
wherein said calcium-chelating substance is selected from citrate or a salt of citrate and is present in a concentration of 55mM to 260mM.

2. A device for collecting a sample comprising a composition according to claim 1 preferably wherein the device is a blood collection tube or blood collection bag

3. A kit of parts comprising:

   - a device according to claim 2, and
   - a reagent for determining blood clotting activity, preferably for determining the development of thrombin activity as a function of time.

4. Use of a thermostable peptide of at most 42 amino acids which inhibits contact activation of the blood clotting system, said peptide comprising the consensus amino acid sequence: RVCPXILMXCKKDSDCLAECXCLEHGYCG (SEQ ID NO: 7), wherein X at position 5 is K or R or an analog of R, preferably R or an analog of R; X at position 9 is E or K, preferably K; and X at position 21 is I or V, preferably V wherein an analog of Arg is selected from the group consisting of: monomethylarginine, symmetric and asymmetric dimethylarginine, canaline, δ-guanidino α-amino butyric acid, homoarginine, canavanine, and citrulline,
for *ex vivo* inhibiting activation of the blood clotting system through contact with foreign surfaces.

5. An *ex vivo* method for inhibiting clotting of blood, the method comprising contacting a thermostable peptide of at most 42 amino acids which inhibits contact activation of the blood clotting system, said peptide comprising the consensus amino acid sequence: RVCPXILMXCKKDSDCLAECXCLEHGYCG (SEQ ID NO: 7), wherein X at position 5 is K or R or an analog of R, preferably R or an analog of R; X at position 9 is E or K, preferably K; and X at position 21 is I or V, preferably V, wherein an analog of Arg is selected from the group consisting of: monomethylarginine, symmetric and asymmetric dimethylarginine, canaline, δ-guanidino α-amino butyric acid, homoarginine, canavanine, and citrullin,
with blood, plasma or any other biological fluid in an amount sufficient to inhibit the clotting.

6.  A composition according to claim 1, a device according to claim 2, a kit according to claim 3, a use according to claim 4, or a method according to claim 5, wherein the thermostable peptide which inhibits contact activation of the blood clotting system, comprises a peptide selected from the group consisting of:

    - a peptide with the sequence as shown in SEQ ID NO: 1,
    - a peptide with the sequence as shown in SEQ ID NO: 2,
    - a peptide with the sequence as shown in SEQ ID NO: 3, and,
    - a peptide with the sequence as shown in SEQ ID NO: 4.

7.  A composition according to claim 1, a device according to claim 2, a kit according to claim 3, a use according to claim 4, or a method according to claim 5, wherein the thermostable peptide which inhibits contact activation of the blood clotting system, is a peptide selected from the group consisting of:

    - a peptide with the sequence as shown in SEQ ID NO: 1,
    - a peptide with the sequence as shown in SEQ ID NO: 2,
    - a peptide with the sequence as shown in SEQ ID NO: 3, and
    - a peptide with the sequence as shown in SEQ ID NO: 4.

**Patentansprüche**

1.  Zusammensetzung, die mindestens eine Calciumchelat bildende Substanz mit Blutgerinnungshemmungsaktivität aufweist und ferner ein thermostabiles Peptid von höchstens 42 Aminosäuren aufweist, welches die Kontaktaktivierung des Blutgerinnungssystems hemmt bzw. inhibiert, wobei das Peptid folgende Aminosäure-Konsensussequenz aufweist:

    RVCPXILMXCKKDSDCLAECXCLEHGYCG (SEQ ID NO: 7), wobei X an Position 5 K oder R oder ein Analogon von R ist, vorzugsweise R oder ein Analogon von R; wobei X an Position 9 E oder K ist, vorzugsweise K; und wobei X an Position 21 I oder V ist, vorzugsweise V, wobei ein Analogon von Arg aus der Gruppe ausgewählt ist, die aus Folgenden besteht: Monomethylarginin, symmetrisches und asymmetrisches Dimethylarginin, Canalin, δ-Guanidino-α-Aminobuttersäure, Homoarginin, Canavanin und Citrullin, und wobei die Calciumchelat bildende Substanz aus Citrat oder einem Salz von Citrat ausgewählt ist und in einer Konzentration von 55 mM bis 260 mM vorhanden ist.

2.  Vorrichtung zum Sammeln einer Probe, die eine Zusammensetzung nach Anspruch 1 aufweist, wobei die Vorrichtung vorzugsweise eine Blutentnahmeröhrchen oder Blutentnahmebeutel ist.

3.  Baukastensystem, das Folgendes aufweist:

    - eine Vorrichtung nach Anspruch 2, und
    - einen Reagenzstoff zum Bestimmen der Blutgerinnungsaktivität, vorzugsweise zum Bestimmen der Entwicklung der Thrombinaktivität als eine Funktion der Zeit.

4.  Verwendung eines thermostabilen Peptids von höchstens 42 Aminosäuren, welches die Kontaktaktivierung des Blutgerinnungssystems hemmt bzw. inhibiert, wobei das Peptid folgende Aminosäure-Konsensussequenz aufweist:

    RVCPXILMXCKKDSDCLAECXCLEHGYCG (SEQ ID NO: 7), wobei X an Position 5 K oder R oder ein Analogon von R ist, vorzugsweise R oder ein Analogon von R; wobei X an Position 9 E oder K ist, vorzugsweise K; und wobei X an Position 21 I oder V ist, vorzugsweise V. wobei ein Analogon von Arg aus der Gruppe ausgewählt ist, die aus Folgenden besteht: Monomethylarginin, symmetrisches und asymmetrisches Dimethylarginin, Canalin, δ-Guanidino-α-Aminobuttersäure, Homoarginin, Canavanin und Citrullin, zur ex vivo Hemmungsaktivierung des Blutgerinnungssystems durch den Kontakt mit fremden Oberflächen.

5. Ex Vivo Verfahren zum Hemmen der Blutgerinnung, wobei das Verfahren das Kontaktieren eines thermostabilen Peptids von höchstens 42 Aminosäuren aufweist, welches die Kontaktaktivierung des Blutgerinnungssystems hemmt bzw. inhibiert, wobei das Peptid folgende Aminosäure-Konsensussequenz aufweist:

RVCPXILMXCKKDSDCLAECXCLEHGYCG (SEQ ID NO: 7), wobei X an Position 5 K oder R oder ein Analogon von R ist, vorzugsweise R oder ein Analogon von R; wobei X an Position 9 E oder K ist, vorzugsweise K; und wobei X an Position 21 I oder V ist, vorzugsweise V. wobei ein Analogon von Arg aus der Gruppe ausgewählt ist, die aus Folgenden besteht: Monomethylarginin, symmetrisches und asymmetrisches Dimethylarginin, Canalin, $\delta$-Guanidino-$\alpha$-Aminobuttersäure, Homoarginin, Canavanin und Citrullin, mit Blut, Plasma oder irgendeiner anderen biologischen Flüssigkeit in einer Menge, die ausreicht, um die Gerinnung zu inhibieren.

6. Zusammensetzung nach Anspruch 1, eine Vorrichtung nach Anspruch 2 ein Baukastensystem nach Anspruch 3, eine Verwendung nach Anspruch 4 oder ein Verfahren nach Anspruch 5, wobei das thermostabile Peptid, das die Kontaktaktivierung des Blutgerinnungssystems hemmt, ein Peptid aufweist, das aus der Gruppe ausgewählt ist, die aus Folgenden besteht:

- einem Peptid mit der in SEQ ID NO: 1 gezeigten Sequenz,
- ein Peptid mit der in SEQ ID NO: 2 gezeigten Sequenz,
- ein Peptid mit der in SEQ ID NO: 3 gezeigten Sequenz, und
- ein Peptid mit der in SEQ ID NO: 4 gezeigten Sequenz.

7. Zusammensetzung nach Anspruch 1, eine Vorrichtung nach Anspruch 2 ein Baukastensystem nach Anspruch 3, eine Verwendung nach Anspruch 4 oder ein Verfahren nach Anspruch 5, wobei das thermostabile Peptid, das die Kontaktaktivierung des Blutgerinnungssystems hemmt, ein Peptid ist, das aus der Gruppe ausgewählt ist, die aus Folgenden besteht:

- einem Peptid mit der in SEQ ID NO: 1 gezeigten Sequenz,
- ein Peptid mit der in SEQ ID NO: 2 gezeigten Sequenz,
- ein Peptid mit der in SEQ ID NO: 3 gezeigten Sequenz, und
- ein Peptid mit der in SEQ ID NO: 4 gezeigten Sequenz.

**Revendications**

1. Composition comprenant au moins une substance chélatrice du calcium avec une activité d'inhibition de la coagulation du sang et comprenant en outre un peptide thermostable d'au moins 42 acides aminés qui inhibe l'activation par contact du système de coagulation du sang, ledit peptide comprenant la séquence consensus d'acides aminés :

RVCPXILMXCKKDSDCLAECXCLEHGYCG (SEQ ID NO : 7), dans laquelle le X en position 5 correspond à K ou R ou à un analogue de R, de préférence à R ou à un analogue de R ; le X en position 9 correspond à E ou K, de préférence à K ; et le X en position 21 correspond à I ou V, de préférence à V, dans laquelle un analogue d'Arg est choisi dans le groupe constitué de: la monométhylarginine, la diméthylarginine symétrique et asymétrique, la canaline, l'acide $\delta$-guanidino $\alpha$-amino butyrique, l'homoarginine, la canavanine et la citrulline, et dans laquelle ladite substance chélatrice du calcium est choisie parmi le citrate ou un sel de citrate et est présente selon une concentration de 55 mM à 260 mM.

2. Dispositif de prélèvement d'un échantillon comprenant une composition selon la revendication 1, de préférence dans lequel le dispositif est un tube de prélèvement sanguin ou une poche de prélèvement sanguin.

3. Ensemble de pièces comprenant :

- un dispositif selon la revendication 2, et
- un réactif de détermination de l'activité de coagulation du sang, de préférence de détermination du développement de l'activité de la thrombine en fonction du temps.

4. Utilisation d'un peptide thermostable d'au moins 42 acides aminés qui inhibe l'activation par contact du système de coagulation du sang, ledit peptide comprenant la séquence consensus d'acides aminés :

RVCPXILMXCKKDSDCLAECXCLEHGYCG (SEQ ID NO : 7), dans laquelle le X en position 5 correspond à K ou R ou à un analogue de R, de préférence à R ou à un analogue de R ; le X en position 9 correspond à E ou K, de préférence à K ; et le X en position 21 correspond à I ou V, de préférence à V, dans laquelle un analogue d'Arg est choisi dans le groupe constitué de: la monométhylarginine, la diméthylarginine symétrique et asymétrique, la canaline, l'acide δ-guanidino α-amino butyrique, l'homoarginine, la canavanine et la citrulline, pour inhiber *ex vivo* l'activation du système de coagulation du sang par contact avec des surfaces étrangères.

5. Procédé *ex vivo* d'inhibition de la coagulation du sang, le procédé comprenant la mise en contact d'un peptide thermostable d'au moins 42 acides aminés qui inhibe l'activation par contact du système de coagulation du sang, ledit peptide comprenant la séquence consensus d'acides aminés :

RVCPXILMXCKKDSDCLAECXCLEHGYCG (SEQ ID NO : 7), dans laquelle le X en position 5 correspond à K ou R ou à un analogue de R, de préférence à R ou à un analogue de R ; le X en position 9 correspond à E ou K, de préférence à K ; et le X en position 21 correspond à I ou V, de préférence à V, dans laquelle un analogue d'Arg est choisi dans le groupe constitué de: la monométhylarginine, la diméthylarginine symétrique et asymétrique, la canaline, l'acide δ-guanidino α-amino butyrique, l'homoarginine, la canavanine et la citrulline, avec du sang, du plasma ou tout autre fluide biologique en quantité suffisante pour inhiber la coagulation.

6. Composition selon la revendication 1, dispositif selon la revendication 2, ensemble selon la revendication 3, utilisation selon la revendication 4 ou procédé selon la revendication 5, dans lesquels le peptide thermostable qui inhibe l'activation par contact du système de coagulation du sang comprend un peptide choisi dans le groupe constitué :

- d'un peptide présentant la séquence définie par SEQ ID NO : 1,
- d'un peptide présentant la séquence définie par SEQ ID NO : 2,
- d'un peptide présentant la séquence définie par SEQ ID NO : 3, et
- d'un peptide présentant la séquence définie par SEQ ID NO : 4.

7. Composition selon la revendication 1, dispositif selon la revendication 2, ensemble selon la revendication 3, utilisation selon la revendication 4, ou procédé selon la revendication 5, dans lesquels le peptide thermostable qui inhibe l'activation par contact du système de coagulation du sang est un peptide choisi dans le groupe constitué :

- d'un peptide présentant la séquence définie par SEQ ID NO : 1,
- d'un peptide présentant la séquence définie par SEQ ID NO : 2,
- d'un peptide présentant la séquence définie par SEQ ID NO : 3, et
- d'un peptide présentant la séquence définie par SEQ ID NO : 4.

*Fig. 1*

*Fig. 2*

*Fig. 3a*

*Fig. 3b*

## Fig. 4a

15.77
TICA-oxidized

18.86
TICA-reduced

0    5    10    15    20    25  minutes 30

## Fig. 4b

$MH^{4+}$
819.5

$MH^{3+}$
1092.3

Mw = 3274.08 ± 0.27

$MH^{2+}$
1638.2

800    1000    1200    1400    1600    1800    2000    2200    2400

m/z,

# Fig. 4c

MH⁴⁺
817.8

MH³⁺
1090.1

**Mw = 3267.68 ± 0.79**

MH²⁺
1635.3

800  1000  1200  1400  1600  1800  2000  2200  2400

m/z,

## Fig. 5

**3 disulfides:**

C3-C20
C10-C22
C16-C28
<u>100% act.</u>

| | 10 | 20 |
|---|---|---|
| RVCPRILMKC | KKDSDCLAEC | VCLEHGYCG |

3-20
10-22
16-28

**2 disulfides:**

C3-C20
C10-C22
<u>0.2% act.</u>

C3-C20
C16-C28
<u>6.8% act.</u>

C10-C22
C16-C28
<u>0.3% act.</u>

**1 disulfides:**

C3-C20
<u>0.003% act.</u>

C10-C22
<u>0.003% act.</u>

C16-C28
<u>0.006% act.</u>

**0 disulfides:**

No C-C
<u>0% act.</u>

EP 2 748 616 B1

# Fig. 6

```
AVCPRILMKC  KKDSDCLAEC  VCLEHGYCG    7.5%
RACPRILMKC  KKDSDCLAEC  VCLEHGYCG    5.9%
RVCARILMKC  KKDSDCLAEC  VCLEHGYCG    9.0%
RVCPAILMKC  KKDSDCLAEC  VCLEHGYCG    0.009%  ←
RVCPRALMKC  KKDSDCLAEC  VCLEHGYCG    23%
RVCPRIAMKC  KKDSDCLAEC  VCLEHGYCG    25%
RVCPRILAKC  KKDSDCLAEC  VCLEHGYCG    38%
RVCPRILMAC  KKDSDCLAEC  VCLEHGYCG    88%
RVCPRILMKC  AKDSDCLAEC  VCLEHGYCG    135%
RVCPRILMKC  KADSDCLAEC  VCLEHGYCG    139%
RVCPRILMKC  KKASDCLAEC  VCLEHGYCG    130%
RVCPRILMKC  KKDADCLAEC  VCLEHGYCG    168%
RVCPRILMKC  KKDSACLAEC  VCLEHGYCG    180%
RVCPRILMKC  KKDSDCAAEC  VCLEHGYCG    18%
RVCPRILMKC  KKDSDCLAEC  VCLEHGYCG    100% TICA
RVCPRILMKC  KKDSDCLAAC  VCLEHGYCG    57%
RVCPRILMKC  KKDSDCLAEC  ACLEHGYCG    23%
RVCPRILMKC  KKDSDCLAEC  VCAEHGYCG    187%
RVCPRILMKC  KKDSDCLAEC  VCLAHGYCG    76%
RVCPRILMKC  KKDSDCLAEC  VCLEAGYCG    160%
RVCPRILMKC  KKDSDCLAEC  VCLEHAYCG    203%
RVCPRILMKC  KKDSDCLAEC  VCLEHGACG    149%
RVCPRILMKC  KKDSDCLAEC  VCLEHGYCA    130%
```

```
         *     10          20
      RVCPRILMKC  KKDSDCLAEC  VCLEHGYCG
          └──┐  │└──┐     │ │         │
          3-20  └───┼──┐  │ │         │
                10-22  └──┘ │         │
                       16-28└─────────┘
```

## REFERENCES CITED IN THE DESCRIPTION

### Patent documents cited in the description

- US 6403381 B
- WO 2003093831 A
- WO 2006117246 A
- WO 2010016762 A

### Non-patent literature cited in the description

- **SCHENONE M ; FURIE BC ; FURIE B.** The blood coagulation cascade. *Current Opinion in Haematology,* 2004, vol. 11 (4), 272-277
- Computational Molecular Biology. Oxford University Press, 1988
- Biocomputing: Informatics and Genome Projects. Academic Press, 1993
- Computer Analysis of Sequence Data. Humana Press, 1994
- **HEINE, G.** Sequence Analysis in Molecular Biology. Academic Press, 1987
- Sequence Analysis. M Stockton Press, 1991
- **CARILLO, H. ; LIPMAN, D. ; SIAM J.** *Applied Math.,* 1988, vol. 48, 1073
- **DEVEREUX, J. et al.** *Nucleic Acids Research,* 1984, vol. 12 (1), 387
- **ALTSCHUL, S. F. et al.** *J. Mol. Biol.,* 1990, vol. 215, 403-410
- **NEEDLEMAN ; WUNSCH.** *J. Mol. Biol.,* 1970, vol. 48, 443-453
- **HENTIKOFF ; HENTIKOFF.** *Proc. Natl. Acad. Sci. USA.,* 1992, vol. 89, 10915-10919
- **NEEDLEMAN, S. B. ; WUNSCH, C. D.** *J. Mol. Biol.,* 1970, vol. 48, 443-453
- An overview of sequence comparison. **KRUSKAL, J. B.** Time warps, string edits and macromolecules: the theory and practice of sequence comparison. Addison Wesley, 1983, 1-44
- **L.POLLER.** Laboratory techniques in thrombosis. 1999, 37-44
- **SAMBROOK et al.** Molecular Cloning, A Laboratory Manual. Cold Spring Harbor Laboratory Press, 1989
- **SAMBROOK ; RUSSELL.** Molecular Cloning: A Laboratory Manual. Cold Spring Harbor Laboratory Press, 2001
- **AUSUBEL et al.** Current Protocols in Molecular Biology. *Current Protocols,* 1994, vol. 1 and 2
- **BROWN.** Molecular Biology LabFax. Academic Press, 1998, vol. I and II
- Laboratory techniques in thrombosis. 1999
- Haemostasis and thrombosis: Basic principles and clinical practice. 2010
- **SCHENONE M ; FURIE BC ; FURIE B.** The blood coagulation cascade. *Current Opinion in Haematology,* 2004, vol. 11 (4), 272-277

SEQUENCE LISTING

<110> Synapse B.V. Universiteit Maastricht

<120> Thermostable inhibitors of activation of the blood clotting system through contact with foreign surfaces

<130> P6036535PCT

<160> 25

<170> PatentIn version 3.3

<210> 1
<211> 29
<212> PRT
<213> Artificial

<220>
<223> Peptide

<400> 1

```
        Arg Val Cys Pro Arg Ile Leu Met Lys Cys Lys Lys Asp Ser Asp Cys
        1               5                   10                  15


        Leu Ala Glu Cys Val Cys Leu Glu His Gly Tyr Cys Gly
                    20                  25
```

<210> 2
<211> 29
<212> PRT
<213> Artificial

<220>
<223> Peptide

<400> 2

Arg Val Cys Pro Arg Ile Leu Met Glu Cys Lys Lys Asp Ser Asp Cys
1               5                   10                  15

Leu Ala Glu Cys Val Cys Leu Glu His Gly Tyr Cys Gly
            20                  25

<210> 3
<211> 32
<212> PRT
<213> Artificial

<220>
<223> Peptide

<400> 3

His Glu Glu Arg Val Cys Pro Arg Ile Leu Met Lys Cys Lys Lys Asp
1               5                   10                  15

Ser Asp Cys Leu Ala Glu Cys Val Cys Leu Glu His Gly Tyr Cys Gly
            20                  25                  30

<210> 4
<211> 29
<212> PRT
<213> Artificial

<220>
<223> Peptide

<400> 4

Arg Val Cys Pro Lys Ile Leu Met Glu Cys Lys Lys Asp Ser Asp Cys
1               5                   10                  15

Leu Ala Glu Cys Ile Cys Leu Glu His Gly Tyr Cys Gly
            20                  25

<210> 5
<211> 32
<212> PRT
<213> Artificial

<220>
<223> Peptide

<400> 5

```
His Glu Glu Arg Val Cys Pro Lys Ile Leu Met Glu Cys Lys Lys Asp
1               5                   10                  15

Ser Asp Cys Leu Ala Glu Cys Ile Cys Leu Glu His Gly Tyr Cys Gly
            20                  25                  30
```

<210> 6
<211> 17
<212> PRT
<213> Artificial

<220>
<223> Peptide

<220>
<221> misc_feature
<222> (1)..(1)
<223> Xaa at position 1 is K or R, preferably R

<220>
<221> misc_feature
<222> (5)..(5)
<223> Xaa at position 5 is E or K, preferably K

<220>
<221> misc_feature
<222> (17)..(17)
<223> Xaa at position 17 is I or V, preferably V

<400> 6

```
Xaa Ile Leu Met Xaa Cys Lys Lys Asp Ser Asp Cys Leu Ala Glu Cys
1               5                   10                  15

Xaa
```

<210> 7
<211> 29
<212> PRT
<213> Artificial

<220>
<223> Peptide

<220>
<221> misc_feature
<222> (5)..(5)
<223> Xaa at position 5 is K or R, preferably R

<220>
<221> misc_feature
<222> (9)..(9)
<223> Xaa at position 9 is E or K, preferably K

<220>
<221> misc_feature

<222> (21)..(21)
<223> Xaa at position 21 is I or V, preferably V

<400> 7

```
        Arg Val Cys Pro Xaa Ile Leu Met Xaa Cys Lys Lys Asp Ser Asp Cys
        1               5                   10                  15

        Leu Ala Glu Cys Xaa Cys Leu Glu His Gly Tyr Cys Gly
                        20                  25
```

<210> 8
<211> 39
<212> PRT
<213> Artificial

<220>
<223> Peptide

<400> 8

```
        Arg Val Cys Gly Ile Gly Pro Arg Pro Arg Leu Pro Trp Pro Arg Ile
        1               5                   10                  15

        Leu Met Lys Cys Lys Lys Asp Ser Asp Cys Leu Ala Glu Cys Val Cys
                        20                  25                  30

                        Leu Glu His Gly Tyr Cys Gly
                                    35
```

<210> 9
<211> 29
<212> PRT
<213> Artificial

<220>
<223> Peptide

<220>
<221> misc_feature
<222> (1)..(2)
<223> Xaa can be any naturally occurring amino acid

<220>
<221> misc_feature
<222> (4)..(4)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)

<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 9

```
        Xaa Xaa Cys Xaa Xaa Xaa Xaa Xaa Xaa Cys Xaa Xaa Xaa Xaa Xaa Cys
        1               5                   10                  15

        Xaa Xaa Xaa Cys Xaa Cys Xaa Xaa Xaa Xaa Xaa Cys Xaa
                    20          25
```

<210> 10
<211> 29
<212> PRT
<213> Artificial

<220>
<223> Peptide

<220>
<221> misc_feature
<222> (2)..(2)
<223> Xaa can be any naturally occurring amino acid

<220>
<221> misc_feature
<222> (4)..(4)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29).. (29)
<223> Xaa can be any naturally occurring amino acid

<400> 10

```
Arg Xaa Cys Xaa Xaa Xaa Xaa Xaa Xaa Cys Xaa Xaa Xaa Xaa Xaa Cys
1               5                   10                  15

Xaa Xaa Xaa Cys Xaa Cys Xaa Xaa Xaa Xaa Xaa Cys Xaa
            20      25
```

<210> 11
<211> 29
<212> PRT
<213> Artificial

<220>
<223> Peptide

<220>

<221> misc_feature
<222> (1)..(1)
<223> Xaa can be any naturally occurring amino acid

<220>
<221> misc_feature
<222> (4)..(4)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 11

```
Xaa Val Cys Xaa Xaa Xaa Xaa Xaa Xaa Cys Xaa Xaa Xaa Xaa Xaa Cys
1               5                   10                  15

Xaa Xaa Xaa Cys Xaa Cys Xaa Xaa Xaa Xaa Xaa Cys Xaa
            20          25
```

<210> 12
<211> 29
<212> PRT
<213> Artificial

<220>
<223> Peptide

<220>
<221> misc_feature
<222> (1)..(2)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)

<223> Xaa can be any naturally occurring amino acid

<400> 12

```
Xaa Xaa Cys Pro Xaa Xaa Xaa Xaa Xaa Cys Xaa Xaa Xaa Xaa Xaa Cys
1               5                   10                  15


Xaa Xaa Xaa Cys Xaa Cys Xaa Xaa Xaa Xaa Xaa Cys Xaa
            20          25
```

<210> 13
<211> 29
<212> PRT
<213> Artificial

<220>
<223> Peptide

<220>
<221> misc_feature
<222> (1)..(2)
<223> Xaa can be any naturally occurring amino acid

<220>
<221> misc_feature
<222> (4)..(4)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 13

```
Xaa Xaa Cys Xaa Arg Xaa Xaa Xaa Xaa Cys Xaa Xaa Xaa Xaa Xaa Cys
1               5                   10                      15

Xaa Xaa Xaa Cys Xaa Cys Xaa Xaa Xaa Xaa Xaa Cys Xaa
            20          25
```

<210> 14
<211> 29
<212> PRT
<213> Artificial

<220>
<223> Peptide

<220>
<221> misc_feature
<222> (4)..(4)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>

<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 14

```
Arg Val Cys Xaa Xaa Xaa Xaa Xaa Xaa Cys Xaa Xaa Xaa Xaa Xaa Cys
1               5                   10                  15


Xaa Xaa Xaa Cys Xaa Cys Xaa Xaa Xaa Xaa Xaa Cys Xaa
            20          25
```

<210> 15
<211> 29
<212> PRT
<213> Artificial

<220>
<223> Peptide

<220>
<221> misc_feature
<222> (2)..(2)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE

<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 15

```
     Arg Xaa Cys Pro Xaa Xaa Xaa Xaa Xaa Cys Xaa Xaa Xaa Xaa Xaa Cys
     1               5                   10                  15


     Xaa Xaa Xaa Cys Xaa Cys Xaa Xaa Xaa Xaa Xaa Cys Xaa
                 20              25
```

<210> 16
<211> 29
<212> PRT
<213> Artificial

<220>
<223> Peptide

<220>
<221> misc_feature
<222> (2)..(2)
<223> Xaa can be any naturally occurring amino acid

<220>
<221> misc_feature
<222> (4)..(4)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)

<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 16

```
Arg Xaa Cys Xaa Arg Xaa Xaa Xaa Xaa Cys Xaa Xaa Xaa Xaa Xaa Cys
1               5                   10                  15
```

```
Xaa Xaa Xaa Cys Xaa Cys Xaa Xaa Xaa Xaa Xaa Cys Xaa
            20                  25
```

<210> 17
<211> 29
<212> PRT
<213> Artificial

<220>
<223> Peptide

<220>
<221> misc_feature
<222> (1)..(1)
<223> Xaa can be any naturally occurring amino acid

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 17

```
Xaa Val Cys Pro Xaa Xaa Xaa Xaa Xaa Cys Xaa Xaa Xaa Xaa Xaa Cys
1               5               10                  15

Xaa Xaa Xaa Cys Xaa Cys Xaa Xaa Xaa Xaa Xaa Cys Xaa
        20          25
```

<210> 18
<211> 29
<212> PRT
<213> Artificial

<220>
<223> Peptide

<220>
<221> misc_feature
<222> (1)..(1)
<223> Xaa can be any naturally occurring amino acid

<220>
<221> misc_feature
<222> (4)..(4)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>

<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 18

```
Xaa Val Cys Xaa Arg Xaa Xaa Xaa Xaa Cys Xaa Xaa Xaa Xaa Xaa Cys
1               5                   10                  15

Xaa Xaa Xaa Cys Xaa Cys Xaa Xaa Xaa Xaa Xaa Cys Xaa
            20          25
```

<210> 19
<211> 29
<212> PRT
<213> Artificial

<220>
<223> Peptide

<220>
<221> misc_feature
<222> (1)..(2)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature

<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 19

```
        Xaa Xaa Cys Pro Arg Xaa Xaa Xaa Xaa Cys Xaa Xaa Xaa Xaa Xaa Cys
        1               5                   10                      15

        Xaa Xaa Xaa Cys Xaa Cys Xaa Xaa Xaa Xaa Xaa Cys Xaa
                    20          25
```

<210> 20
<211> 29
<212> PRT
<213> Artificial

<220>
<223> Peptide

<220>
<221> misc_feature
<222> (2)..(2)
<223> Xaa can be any naturally occurring amino acid

<220>
<221> misc_feature
<222> (4)..(4)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)

<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 20

```
        Arg Xaa Cys Xaa Arg Xaa Xaa Xaa Xaa Cys Xaa Xaa Xaa Xaa Xaa Cys
        1               5                   10                  15


        Xaa Xaa Xaa Cys Xaa Cys Xaa Xaa Xaa Xaa Xaa Cys Xaa
                    20              25
```

<210> 21
<211> 29
<212> PRT
<213> Artificial

<220>
<223> Peptide

<220>
<221> MISC_FEATURE
<222> (5)..(5)
<223> Xaa at position 5 is R or an analog of R

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 21

```
Arg Val Cys Pro Xaa Xaa Xaa Xaa Xaa Cys Xaa Xaa Xaa Xaa Xaa Cys
1               5                   10                  15

Xaa Xaa Xaa Cys Xaa Cys Xaa Xaa Xaa Xaa Xaa Cys Xaa
            20          25
```

<210> 22
<211> 29
<212> PRT
<213> Artificial

<220>
<223> Peptide

<220>
<221> misc_feature
<222> (4)..(4)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>

<221> misc_feature
<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 22

```
Arg Val Cys Xaa Arg Xaa Xaa Xaa Xaa Cys Xaa Xaa Xaa Xaa Xaa Cys
1               5                   10                  15

Xaa Xaa Xaa Cys Xaa Cys Xaa Xaa Xaa Xaa Xaa Cys Xaa
            20          25
```

<210> 23
<211> 29
<212> PRT
<213> Artificial

<220>
<223> Peptide

<220>
<221> misc_feature
<222> (1)..(1)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature

<222> (11)..(15)
<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 23

```
Xaa Val Cys Pro Arg Xaa Xaa Xaa Xaa Cys Xaa Xaa Xaa Xaa Xaa Cys
1               5               10                      15

Xaa Xaa Xaa Cys Xaa Cys Xaa Xaa Xaa Xaa Xaa Cys Xaa
            20          25
```

<210> 24
<211> 29
<212> PRT
<213> Artificial

<220>
<223> Peptide

<220>
<221> misc_feature
<222> (2)..(2)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)

<223> Xaa can be any naturally occurring amino acid

<220>
<221> misc_feature
<222> (17)..(19)
<223> Xaa can be any naturally occurring amino acid

<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29).. (29)
<223> Xaa can be any naturally occurring amino acid

<400> 24

```
        Arg Xaa Cys Pro Arg Xaa Xaa Xaa Xaa Cys Xaa Xaa Xaa Xaa Xaa Cys
        1               5                   10                  15


        Xaa Xaa Xaa Cys Xaa Cys Xaa Xaa Xaa Xaa Xaa Cys Xaa
                    20          25
```

<210> 25
<211> 29
<212> PRT
<213> Artificial

<220>
<223> Peptide

<220>
<221> misc_feature
<222> (6)..(8)
<223> Xaa can be any naturally occurring amino acid

<220>
<221> MISC_FEATURE
<222> (9)..(9)
<223> Xaa at position 9 is E or K, preferably K

<220>
<221> misc_feature
<222> (11)..(15)
<223> Xaa can be any naturally occurring amino acid

<220>
<221> misc_feature
<222> (17)..(19)
<223> Xaa can be any naturally occurring amino acid

```
<220>
<221> MISC_FEATURE
<222> (21)..(21)
<223> Xaa at position 21 is I or V, preferably V

<220>
<221> misc_feature
<222> (23)..(27)
<223> Xaa can be any naturally occurring amino acid

<220>
<221> misc_feature
<222> (29)..(29)
<223> Xaa can be any naturally occurring amino acid

<400> 25
```

```
Arg Val Cys Pro Arg Xaa Xaa Xaa Xaa Cys Xaa Xaa Xaa Xaa Xaa Cys
 1               5                  10                  15


Xaa Xaa Xaa Cys Xaa Cys Xaa Xaa Xaa Xaa Xaa Cys Xaa
            20              25
```